# EUROPEAN PATENT SPECIFICATION

(11) **EP 3 426 316 B1**
(45) Date of publication and mention of the grant of the patent: **25.10.2023**
(21) Application number: 17763968.9
(22) Date of filing: 08.03.2017
(51) Int. Cl.: A61L 31/16, A61L 31/04, A61K 9/51, A61K 47/24, A61K 33/00

(54) **DELIVERY OF NITRIC OXIDE-RELEASING PHOSPHOLIPIDS, LIPOSOMES, AND HIGH DENSITY LIPOPROTEIN-LIKE NANOPARTICLES (HDL NPS) BY DRUG ELUTING STENTS AND INTRA-ARTERIAL INJECTION**
VERABREICHUNG VON STICKOXIDFREISETZENDEN PHOSPHOLIPIDEN, LIPOSOMEN UND HOCHDICHTEN LIPOPROTEINÄHNLICHEN NANOPARTIKELN (HDL NPS) DURCH MEDIKAMENTENELUIERENDE STENTS UND INTRA-ARTERIELLE INJEKTION
ADMINISTRATION DE PHOSPHOLIPIDES, DE LIPOSOMES ET DE NANOPARTICULES DE TYPE LIPOPROTÉINES HAUTE DENSITÉ (HDL NPS) LIBÉRANT DE L'OXYDE NITRIQUE FAISANT APPEL À DES ENDOPROTHÈSES À ÉLUTION MÉDICAMENTEUSE ET À L'INJECTION INTRA-ARTÉRIELLE

(30) Priority: 08.03.2016 US 201662305181 P
(43) Date of publication of application: 16.01.2019
(73) Proprietor: Northwestern University, Evanston, IL 60208 (US); Nanyang Technological University, Singapore 639798 (SG)
(72) Inventor: THAXTON, C., Shad, Chicago, IL 60614 (US); RINK, Jonathan, Park Ridge, IL 60068 (US); VENKATRAMAN, Subbu, Singapore 639798 (SG); HUANG, Yingying, Singapore Republic of Singapore 63978 (SG)
(74) Representative: Hoffmann Eitle
(86) International application number: PCT/US2017/021293
(87) International publication number: WO 2017/156078

(56) References cited:
- WO-A1-01/03709
- WO-A1-03/017989
- WO-A1-2005/068020
- WO-A2-2009/131704
- WO-A2-2010/137037
- US-A1- 2008 175 893
- US-A1- 2008 181 928
- US-A1- 2011 052 680
- US-A1- 2015 182 543
- NOORA NAGHAVI ET AL: "Nitric Oxide Donors for Cardiovascular Implant Applications", SMALL, vol. 9, no. 1, 14 January 2013 (2013-01-14), pages 22-35, XP055333206, DOI: 10.1002/smll.201200458

## Description

### RELATED APPLICATIONS

### GOVERNMENT SUPPORT

This invention was made with government support under 5 R01 HL116577 awarded by the National Institutes of Health. The government has certain rights in the invention.

### FIELD OF INVENTION

The present invention generally relates to delivery of nitric oxide nanoparticles, including liposomes, as therapy for disease.

### BACKGROUND

Narrowing of arteries, due to the proliferation and migration of the underlying muscle cells into the blood vessel, is a major complication of any therapeutic intervention taken to open a blocked artery, including balloon angioplasty. Currently, stents, including bare metal and drug loaded variants, are used to reduce the narrowing of the artery post procedure. However, narrowing can still occur with the bare metal stents, while the drug loaded stents have significant side effects associated with them and require patients to take blood thinners for the rest of their lives. Nitric oxide (NO), a highly reactive gas, has been demonstrated to have protective effects on blood vessels, significantly reducing narrowing after intervention as well as promoting the health of the cells lining the blood vessel. NO is extremely difficult to deliver, and currently there are no therapeutics that can deliver NO clinically. Attempts have been made to develop nitric oxide releasing nanoparticles/nanomaterials. In the prior attempts, limitations such as toxicity and instability of the nanomaterials in water/PBS in the materials being used (e.g. peptide amphiphiles, glass nanoparticles) prevented their application to biological systems. A drug eluting stent, loaded with the NO donor nitroprusside, did not reduce neointimal hyperplasia and restenosis in a porcine coronary injury model (Yoon et al. Yonsei Med J. 2002 Apr. 43(2): 242-51).
US 2015/0182543 A1 relates to nitric oxidereleasing particles for delivering nitric oxide, and their use in biomedical and pharmaceutical applications. Among others, it describes an implantable device or injectable composition, or coating on a catheter comprising nitric oxide releasing nanoparticles that can have a gold core. WO 03017989 A1 relates to medical devices comprising NO-donors which include S-Nitrosylated lipids, but devices do not comprise nanostructures.

### SUMMARY OF THE INVENTION

The invention in some aspects is an implantable device comprising a nanostructure of reservoir molecules that are reservoirs for nitric oxide; wherein the reservoir molecule is a S-Nitrosylated lipid or a S-Nitrosylated phospholipid such as S-Nitrosylated 1,2-dipalmitoyl-sn-glycero-3-phosphothioethanol (DPPTE), and wherein the nanostructure has a gold core and a lipid bilayer or monolayer.

Optionally the structure includes an apolipoprotein. In some embodiment is apolipoprotein is apoliprotein A-I (apo A-I).

The lipid shell is a lipid bilayer or a lipid monolayer, the core is a gold core. In embodiments the structure has 60-250 fold excess lipid to gold core. In other embodiments the reservoir molecules contain an NO donating group.

In other aspects the invention is a nanostructure of reservoir molecules that are reservoirs for nitric oxide for use in a method for delivering NO to a subject having a NO mediated disorder, the method comprising: administering by injection, through a catheter or on an implantable device, of the nanostructure of reservoir molecules, wherein the reservoir molecules are a S-Nitrosylated lipid or a S-Nitrosylated phospholipid such as S-Nitrosylated 1,2-dipalmitoyl-sn-glycero-3-phosphothioethanol (DPPTE), and wherein the nanostructure has a gold core and a lipid bilayer or monolayer.

In some embodiments the NO mediated disorder is angiogenesis or ischemia-reperfusion. In some embodiments, the NO mediated disorder is restenosis. In some embodiments, the NO mediated disorder is angioplasty. In some embodiments, the NO mediated disorder is atherosclerosis. In some embodiments, the NO mediated disorder is dysregulated blood pressure. In some embodiments, the NO mediated disorder is graft rejection after transplantation.

In some embodiments, the nanostructure further comprises an apolipoprotein. In some embodiments, the apolipoprotein is apolipoprotein A-I (apoA-I).

In other aspects the invention is a S-Nitrosylated lipid or a S-Nitrosylated phospholipid such as S-Nitrosylated 1,2-dipalmitoyl-sn-glycero-3-phosphothioethanol (DPPTE) for use in a method for transplanting a donor organ in a recipient subject, the method comprising: harvesting a donor organ; contacting the donor organ with the implantable device described herein; and transplanting the donor organ into a recipient subject, wherein the implantable device reduces the risk of rejection of the donor organ relative to the risk of a donor organ transplanted without exposure to the implantable device.

In other aspects the invention is a S-Nitrosylated lipid or a S-Nitrosylated phospholipid such as S-Nitrosylated 1,2-dipalmitoyl-sn-glycero-3-phosphothioethanol (DPPTE) for use in a method for transplanting an organ in a recipient subject, the method comprising: preparing an implantable device that is an organ; wherein the implantable device is a device as described herein; and transplanting the organ into a recipient subject.

The details of one or more embodiments of the invention are set forth in the accompanying Detailed Description, Examples, Claims, and Figures. Other features, objects, and advantages of the invention will be apparent from the description and from the claims.

### BRIEF DESCRIPTION OF THE DRAWINGS

E nbodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
**FIGs. 1A-1D** show the synthesis of S-nitrosylated DPPTE (FIG. 1A). FIGs. 1B-1D show three graphs depicting the value and velocity of the reaction.
**FIGs. 2A-2C**are a set of graphs depicting the characterization of the synthesized S-nitrosylated DPPTE.
**FIGs. 3A-3B** show the synthesis of Nitric Oxide liposomes (FIG. 3A) and HDL NPs (NO-HDL NPs, FIG. 3B).
**FIGs. 4A-4C** show the characterization of NO-HDL NPs. FIG. 4A shows a bar graphing depicting NO release from DPPTE liposomes versus NO liposomes. FIGs. 4B and 4C show the size of DPPTE liposomes (FIG. 4C) versus NO liposomes (FIG. 4B).
**FIGs. 5A-5B** show the characterization of NO-HDL NPs.
**FIG. 6** shows a schematic of the design of drug eleuting stents coated with SNO DPPTE, NO Liposomes, HDL NPs and NO-HDL NPs.
**FIGs. 7A-7B** show a set of graphs demonstrating changes to LPS-induced gene expression with HDL NPs and NO-HDL NPs.
**FIGs. 8A-8D** show the characterization of SNO-PL. (FIG. 8A shows the reaction scheme for production of SNO DPPTE. FIG. 8B shows UV/Vis spectra for DPPTE and SNO-PL, with the S-N=O peak at 335nm. FTIR (FIG. 8C) and Ra-man (FIG. 8D) spectra demonstrate conversion of -SH group of DPPTE to an -S-N=O group in SNO-PL.
**FIGs. 9A-9B****.** show *in vitro* stability, toxicity and efficacy of SNO HDL NPs. FIG. 9A shows the SNO group on SNO HDL NPs was stable when stored at +4oC for up to 50 days before appreciably decreasing. *p<0.05 v. Day 1. FIG. 9B shows toxicity of SNO HDL NPs and HDL NPs on HAEC and AoSMCs. FIG. 9C shows SNO HDL NPs reduce migration of AoSMCs. *p<0.05 v. PBS and SNO HDL NP; **p<0.05 v. PBS and HDL NP.
**FIGs. 10A-10B** show *in vivo* model of kidney transplantation. FIG. 10A shows plasma creatinine levels of mouse kidney transplant recipients on day 2 post transplantation. *p<0.05 v. PBS control. FIG. 10B shows immunocytochemistry for Gr-1 (gray), a neutrophil marker, in representative sections of PBS, HDL NP and SNO HDL NP treated kidney recipients. DAPI stain is also shown.
**FIGs. 11A-11B** show the reaction kinetics and stoichiometry of S-nitrosylation of DPPTE. FIG. 11A shows the phospholipid DPPTE and sodium nitrite were added at various ratios, and S-nitrosylation reaction monitored using a UV/Vis spectrophotometer. FIG. 11B shows mass spectroscopy analysis of phospholipid to nitrite combinations.
**FIG. 12** shows UV/Vis Spectra of HDL NP and SNO HDL NP. UV/Vis spectra of HDL NP and SNO HDL NP constructs demonstrates a local maximum at ~520nm. The SNO peak at 335nm in the SNO HDL NP is not visible due to background signal from the HDL NP.
**FIG. 13** shows representative images of AoSMC transwell migration assay. Representative images of crystal violet stained AoSMC cells following transwell migration.
**FIG. 14** shows TUNEL staining of transplanted kidney grafts on day 2. Representative images of TUNEL staining (PBS; HDL NP and SNO-HDL NP) in transplanted kidney grafts. Nuclei counter stained with DAPI.
**FIG. 15** shows Ki67 staining of transplanted kidney grafts on day 2. Kidney grafts were stained for Ki67, a proliferation marker and nuclei stained with DAPI.

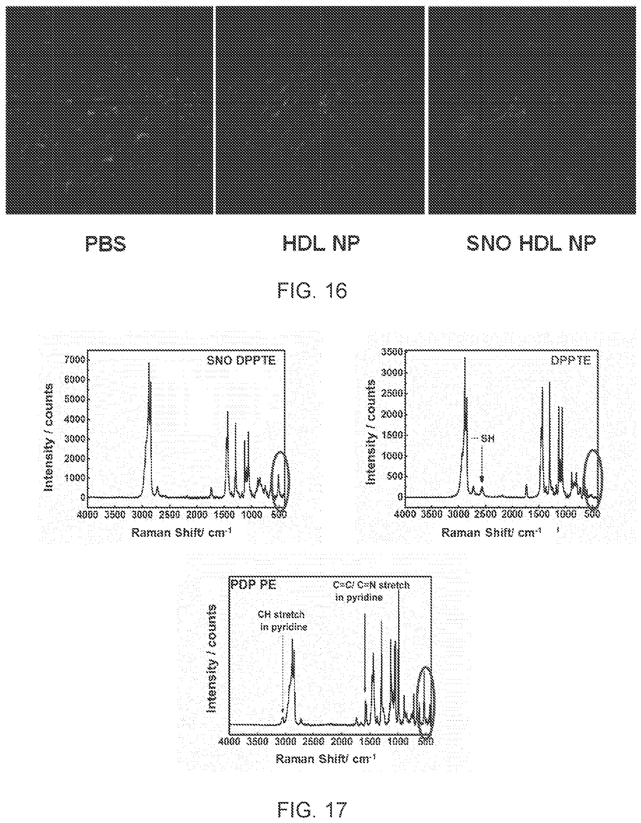

**FIG. 16** shows macrophage staining of transplanted kidney grafts on day 2. Representative images of transplanted kidney grafts stained for F4/80, a macro-phage marker and nuclei stained with DAPI.
**FIG. 17** shows SNO DPPTE forms disulfide bonds when exposed to light and/or room temperature.

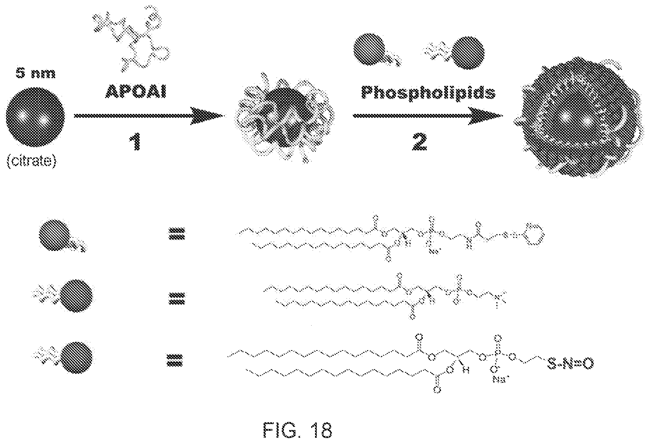

**FIG. 18** shows nitric oxide-releasing HDL NPs (SNO HDL NPs).

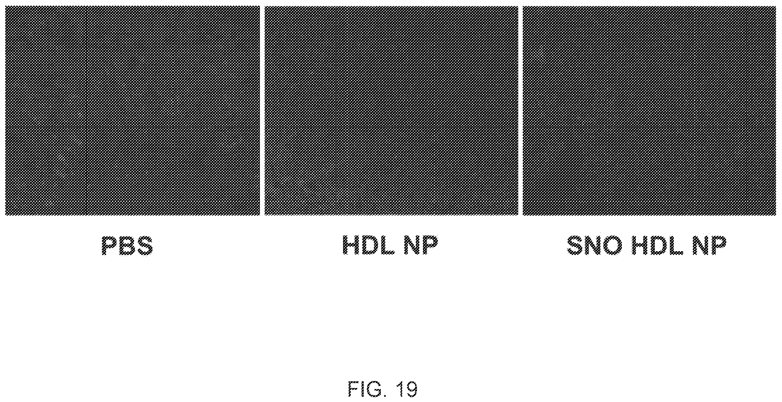

**FIG. 19** shows kidney transplant histology: GR-1 (gray; neutrophils). Nuclei were stained with DAPI.

### DETAILED DESCRIPTION

The invention described herein, in some aspects, is a versatile platform for targeted delivery of nitric oxide, based on synthetic nanostructures such as liposomes and high-density lipoprotein nanoparticles (HDL-NPs). The novel lipid molecules for NO delivery may be directly administered to a subject or incorporated into a medical device or other carrier and implanted into the body. These formulations have utility in angioplasty and stent placement as well as therapies to prevent neointimal hyperplasia and re-stenosis of the vessel while, and promoting endothelial cell growth and migration.

The present invention has several advantages over prior art systems. For instance, a large amount of NO can be released locally from a small amount of material, relative to prior art delivery devices. This is accomplished because S-nitrosylation of phospholipid DPPTE allows for the release of NO from S-nitroso group; following NO release, the S radical can interact with nitrite and/or arginine in serum to 'regenerate' the NO group, providing for additional NO release. A large number of phospholipids in each liposome or nanoparticle translates to the potential for a large bolus of NO to be delivered. Adsorption into DES results in a relatively high NO concentration near the implant but much lower NO concentrations in distal blood vessels. Intra-arterial injection of phospholipid and/or liposomes and/or NO-HDL NPs results in high local NO concentrations at the site of vascular injury but lower or normal NO levels in distal blood vessels.

Modification of the thiol-containing phospholipid DPPTE, through S-nitrosylation, transforms the lipids into nitric oxide reservoirs. In addition, after release of NO, the sulfur radical can react with arginine to regenerate the S-N=O group, thus allowing for sustained nitric oxide release over time. Incorporation of SNO DPPTE into high density lipoprotein nanoparticles (HDL NPs) and liposomes results in novel delivery vehicles for nitric oxide. These nitric oxide reservoirs (NO-HDL NPs, NO Liposomes, SNO DPPTE) as well as HDL NPs can be loaded onto implantable devices, such as bare metal stents, using, for instance, a biodegradable polymer to achieve drug delivery at the site of injury.

Nanostructures include, for instance, liposomes, S-nitrosylation of DPPTE (SNO DPPTE), and NO-HDL NPs. SNO carrying liposomes are referred to herein as NO Liposomes. SNO DPPTE is dried in a glass vial under nitrogen gas to create a thin film. Water is added to the vial, which is then sonicated for 10-30 minutes to produce liposomes of ~30-100nm in diameter.

NO-HDL-NPs are synthesized using a nanoparticle core, such as a gold core, to control size and shape, and modified lipids that harbor nitric oxide and serve as nitric oxide releasing nanoparticles. NO releasing high density lipoprotein nanoparticles have been designed with similar characteristics to natural HDL (the 'good' cholesterol). The NPs in some aspects contain molecules such as phospholipids modified in such a way to release nitric oxide, as well as regenerate their NO group through interaction with the amino acid arginine. These materials may be used as treatment for diseases of cholesterol overload, in instances of revascularization, or as therapy in any case of where ischemia-reperfusion injury is suspected.

In aspects, the present invention generally relates to the prevention of restenosis following vascular interventions (e.g. angioplasty), the reduction of ischemia-reperfusion injury following myocardial infarction and/or organ transplantation, prolonging of cold ischemia time of donor organs, the reduction of atherosclerotic plaque burden, ameliorating endothelial dysfunction and stiffening in atherosclerosis development, and as a therapy for blood pressure.

The present invention has advantages including, S-nitrosylation of the phospholipid in outer leaflet of HDL NPs, which allows the nanoparticles to deliver nitric oxide to locations targeted by HDL NPs (e.g. SR-B 1 expressing cells), improving biomimetic nanoparticle design, stabilizing nanoparticle formulation, and allowing a large number of phospholipids on the outer leaflet of lipid bilayer creating a large number of S-nitrosylated phospholipids per nanoparticle.

High density lipoprotein nanoparticles (HDL NPs) mimic natural spherical HDLs in their shape, size, surface composition (apolipoprotein A1, phospholipids), and ability to functionally efflux cholesterol from cells. Modification of the outer phospholipid, through S-nitrosylation, transforms the lipids into nitric oxide reservoirs. In addition, after release of NO, the sulfur radical can react with arginine to regenerate the S-N=O group, thus potentially allowing for sustained nitric oxide release over time.

High-density lipoproteins (HDL) are naturally-occurring nanoparticles that assemble dynamically in serum from phospholipids, apolipoproteins, and cholesterol. HDL is involved in reverse-cholesterol transport, and has been epidemiologically correlated with reduced incidences of cardiovascular disease (Asztalos, B. F., Tani, M. & Schaefer, E. J.et al. Metabolic and functional relevance of HDL subspecies. Current Opinion in Lipidology 22, 176-185 (2011); Barter, P. et al. HDL cholesterol, very low levels of LDL cholesterol, and cardiovascular events. N. Engl. J. Med. 357, 1301-1310 (2007). Natural HDL is known to bind Scavenger Receptor type B-1 (SR-B 1); SR-B 1 mediates uptake of cholesteryl esters and the uptake and efflux free cholesterol.

Nitric oxide (NO) is a gaseous signaling molecule with fundamental actions in biology with numerous regulatory, protective and therapeutic properties. In higher vertebrates it has key roles in maintaining homeostasis and in smooth muscle (especially vascular smooth muscle), neurons and the gastrointestinal tract. NO is involved in regulating aspects from waking, digestion, sexual function, perception of pain and pleasure, memory recall and sleeping. The way NO functions in the body influences how humans degenerate with age. NO also plays a key role in cardiovascular disease, stroke, diabetes, and cancer. Thus, the ability to control NO signaling and to use NO effectively in therapy presents a major bearing on the future quality and duration of human life.

NO is produced from L-arginine by nitric oxide synthase (hereinafter referred to as NOS). The NOS of the human body has three NOS isomers. The different NOS isoforms exhibit tissue- and cell-type specific distributions and activities, which reflect their specific physiological roles. eNOS is active primarily in the endothelial tissue of blood vessels, where NO mediates vasodilation and relaxation of soft tissue [Moncada S, Bolanos JP. Nitric oxide, cell bioenergetics and neurodegeneration. J Neurochem 2006; 97: 1676-1689]. eNOS is a constitutively active isoform that produces low levels of NO at a steady rate over long periods to achieve its functional roles [Moncada S, Bolanos JP. Nitric oxide, cell bioenergetics and neurodegeneration. J Neurochem 2006; 97: 1676-1689]. iNOS is active primarily in immune cells and glial cells and is activated by pathogen recognition and cytokine release [Moncada S, Bolanos JP. Nitric oxide, cell bioenergetics and neurodegeneration. J Neurochem 2006; 97: 1676-1689; Merrill JE, Murphy SP, Mitrovic B et al. Inducible nitric oxide synthase and nitric oxide production by oligodendrocytes. J Neurosci Res 1997; 48: 372-384]. The primary function of iNOS is to mediate cell death in response to pathogens by generating NO at toxic levels. Thus, iNOS produces high concentrations of NO over short periods (Knott AB, Bossy-Wetzel E. Nitric oxide in health and disease of the nervous system. Antioxid Redox Signal 2009; 11: 541-554). nNOS is active primarily in central and peripheral neurons where NO serves as an important neurotransmitter in cell-to-cell communication and neuronal plasticity [Knott AB, Bossy-Wetzel E. Nitric oxide in health and disease of the nervous system. Antioxid Redox Signal 2009; 11: 541-554]. Similar to eNOS, nNOS is constitutively active and produces low levels of NO over long periods. Finally, mtNOS is the most recently identified member of the NOS family [Ghafourifar P, Cadenas E. Mitochondrial nitric oxide synthase. Trends Pharmacol Sci 2005; 26: 190-195]. mtNOS localizes to the mitochondrial inner membrane and plays a role in the regulation of bioenergetics and Ca2+ buffering [Ghafourifar P, Richter C. Nitric oxide synthase activity in mitochondria. FEBS Lett 1997; 418: 291-296].

NO contributes to various pathologies through formation of reactive nitrogen species (RNS) and modification of proteins and also plays important physiological roles in blood vessel dilation, neurotransmission and immune cell response. NO was first identified as the endothelium-derived relaxing factor that mediates blood vessel dilation [Ignarro LJ, Buga GM, Wood KS, Byrns RE, Chaudhuri G. Endothelium-derived relaxing factor produced and released from artery and vein is nitric oxide. Proc Natl Acad Sci USA 1987; 84: 9265-9269]. In addition, NO is involved in multiple nervous system activities including nerve-mediated relaxation of the gut during digestion [Snyder SH. Nitric oxide: first in a new class of neurotransmitters. Science 1992; 257: 494-496], innervation of neural blood vessels in cerebral and penile arteries [Bredt DS, Glatt CE, Hwang PM, Fotuhi M, Dawson TM, Snyder SH. Nitric oxide synthase protein and mRNA are discretely localized in neuronal populations of the mammalian CNS together with NADPH diaphorase. Neuron 1991; 7: 615-624; Bredt DS, Hwang PM, Glatt CE, Lowenstein C, Reed RR, Snyder SH. Cloned and expressed nitric oxide synthase structurally resembles cytochrome P-450 reductase. Nature 1991; 351: 714-718; Burnett AL, Lowenstein CJ, Bredt DS, Chang TS, Snyder SH. Nitric oxide: a physiologic mediator of penile erection. Science 1992; 257: 401-403] and prevention of excitotoxicity by S-nitrosylation of N-methyl-d-aspartate (NMDA) glutamate receptors [Choi YB, Tenneti L, Le DA et al. Molecular basis of NMDA receptor-coupled ion channel modulation by S-nitrosylation. Nat Neurosci 2000; 3: 15-21; Kim WK, Choi YB, Rayudu PV et al. Attenuation of NMDA receptor activity and neurotoxicity by nitroxyl anion, NO. Neuron 1999; 24: 461-469].

Augmenting the body's natural generation of NO by either stimulating increased production of endogenous NO or introducing exogenously-produced NO into the body can improve the body's response to damage, pain, and invading organisms. However, it is difficult to deliver NO into living tissue. To be clinically useful, NO must be present in the site of action in a sufficient quantity.

Prior methods for delivering NO for therapeutic purposes include the administration of chemical compounds which release NO chemically into the body. Other methods employ NO pathway agonists and NO antagonists. Still other methods employ high pressure NO gas and sprays. Yet another method involves surrounding a body with sealed vacuum containers into which gaseous NO is introduced. Attempts have also been made to force pressurized nitric oxide through tissue and skin. For various reasons, these methods have yielded limited results. For example, gaseous NO is highly reactive, has low diffusion constant and has extremely short life-time in tissue media.

There are several solutions that target specific clinical outcomes involving NO. Sildenafil citrate (sold under the brand name VIAGRA^{®}), for example, interferes with the down regulation of NO in erectile dysfunction syndrome. Etanercept (sold under the brand name ENBRIL), for example, uses an anti-TNF alpha antibody to do what NO would do in inflammatory diseases of the joint. Most solutions involve affecting the NO pathways, due to the difficulty in stimulating production of NO directly at the site of action. Because of the lack of site specificity of these NO pathway pharmacologics, negative side effects can be detrimental.

NO plays an active defense role in the immune system. It is a strong antioxidant, and can suppress bacterial infections, viruses and parasitic attacks. NO can be used to reduce inflammation, facilitate vasodilation, alleviate pain associated with joint swelling in arthritis, including pain associated with osteoarthritis and Rheumatoid Arthritis, combating Gram Positive microorganisms, Gram Negative microorganisms, Fungi (including onychomycosis) and viruses. It is also therapeutic in treating osteoporosis, collagen formation, stem cell signaling, satellite cell differentiation, wound-healing, wound-management, reduction in scar tissue, remediation of activity related injury, and acne. It can even deter some types of cancer cell growth and inhibit cancer cell proliferation. NO can also enhance nerve regeneration, promote apoptosis, stimulate endogenous nitric oxide production, and stimulate iNOS pathways.

NO can effectively function to maintain homeostasis in the cardiovascular and respiratory systems. NO, as a signaling molecule, causes vasodilation which promotes blood vessel flexibility, eases blood pressure, cleans the blood, reverses atherosclerosis and effectively prevents cardiovascular diseases and aids in its recovery. NO slows down atherosclerotic plaque deposition on vascular walls. In patients with moderate to severe diabetes, NO can prevent many common and serious complications. NO can effectively decrease the risk of cancer, diabetes, myocardial infarction and stroke. In the respiratory system, nitric oxide (NO) dilates blood vessels in the lungs, improving oxygenation of the blood and reducing pulmonary hypertension. Because of this, nitric oxide is provided as a therapeutic gas for patients with pulmonary hypertension.

NO can also slow the aging process and improve memory. The NO molecules produced by the immune system are not only capable of destroying invading microorganisms, but also help activate and nourish brain cells, significantly slowing aging and improving memory.

Besides s-nitrosylation (e.g., nitrosylated lipid), another example of a modification to generate a NO-donating group is nitrosylation of a nitrogen (N-nitrosylation) to provide an N-nitrosylated molecule (e.g., a lipid). In some embodiments, the reservoir molecule is a lipid molecule that has been modified to include other molecules that can donate an NO group. Examples of other molecules include diazeniumdiolates (also known as NONOates) (See e.g., Ramamurthi et al. (1997) Chem Res Toxicol 10(4):408-413). Diazeniumdiolates typically have half-lives of milliseconds in biological systems (e.g., cell culture media, plasma,). The reservoir molecule (e.g., nitrosylated lipid) is able to release a NO group at a target site. Examples of non-lipid reservoir molecules, include, glutathione (See e.g., Pompella et al., Biochem Pharmacol 2003 66(8): 1499-1503). Glutathione is a tripeptide that acts as a natural NO reservoir *in vivo.* In some embodiments, the structure, nanostructure or nanoparticle (e.g., HDL nanoparticle) described herein contains one or more glutathiones. In some embodiments, the free thiol in glutathione is modified (e.g., S-nitrosylated).

Other examples of NO donors include L-arginine and L-arginine hydrochloride, D,L-arginine, D-arginine, or alkyl (e.g., ethyl, methyl, propyl, isopropyl, butyl, isobutyl, tert-butyl,) esters of L-arginine and/or D-arginine (e.g., a methyl ester, an ethyl ester, a propyl ester, a butyl ester,) and/or salts thereof, as well as other derivatives of arginine and other NO donors. For instance, examples of pharmaceutically acceptable salts include hydrochloride, glutamate, butyrate, or glycolate (e.g., resulting in L-arginine glutamate, L-arginine butyrate, L-arginine glycolate, D-arginine hydrochloride, D-arginine glutamate, ete.). Other examples of NO donors include L-arginine-based compounds such as, L-homoarginine, N-hydroxy-L-arginine, nitrosylated L-arginine, nitrosylated L-arginine, nitrosylated N-hydroxy-L-arginine, nitrosylated N-hydroxy-L-arginine, citrulline, omithine, linsidomine, nipride, glutamine, and salts thereof (e.g., hydrochloride, glutamate, butyrate, glycolate,). Still other examples of NO donors include S-nitrosothiols, nitrites, 2-hydroxy-2-nitrosohydrazines, or substrates of various forms of NOS. In some cases, the NO may be a compound that stimulates endogenous production of NO *in vivo.* Examples of such compounds include, L-arginine, substrates of various forms of NOS, certain cytokines, adenosine, bradykinin, calreticulin, bisacodyl, phenolphthalein, OH-arginine, or endothelein. It should be understood that, in any of the embodiments described herein that describe a S-nitrosylated lipid, other NO donors may also be used in combination with, S-nitrosylated lipids, in other embodiments of the invention.

The NO reservoir nanoparticles of the invention are coated onto or otherwise incorporated into an implantable device. The term "implantable" refers to the ability to be implanted in a mammal (e.g., a human being or patient) that meets the mechanical, physical, chemical, biological, and pharmacological requirements of a device provided by laws and regulations of a governmental agency (e.g., the U.S. FDA) such that the device is safe and/or effective for use as indicated by the device. Implantable devices may be made from any suitable substrate that can be implanted into a human or non-human animal. Examples of implantable devices include, stents such as self-expandable stents, balloon-expandable stents, coronary stents, peripheral stents, stent-grafts, shunts, catheters, other expandable tubular devices for various bodily lumen or orifices, grafts, vascular grafts, arterio-venous grafts, by-pass grafts, pacemakers and defibrillators, leads and electrodes, screws, valves, such as artificial heart valves, anastomotic clips, arterial closure devices, closure devices, cerebrospinal fluid shunts, and particles (e.g., drug-eluting particles, microparticles and nanoparticles). The implantable devices may be used in any vessel or tissue in the body, including neurological, carotid, vein graft, coronary, aortic, renal, iliac, femoral, popliteal vasculature, and urethral passages.

The device may include a bioabsorbable coating. A method of fabricating the coated implantable device may involve forming one or more layers on surface of the device comprising a polymer such as a high or low molecular weight absorbable polymer. The implantable device including the coating may be degradable or bioabsorbable. In some embodiments, the coating may be degradable or bioabsorbable and can degrade within about 1 month, 2 months, 3 months, 4 months, or 6 months after implantation. The coating may be completely or substantially completely absorbed upon implantation in a human body.

The coating of the invention (nanostructure of reservoir molecules that are reservoirs for nitric oxide) can be added to the implantable device as a coating containing other components such as a partially or completely biodegradable/bioabsorbable/ bioerodable polymer, a biostable polymer, or a combination thereof. A layer or a film (e.g., a coating) may be disposed over an implantable device, such that a coating of the material deposited directly or indirectly over at least a portion of the surface of the device. Direct depositing means that the coating is applied directly to the exposed surface of the device. Indirect depositing means that the coating is applied to an intervening layer that has been deposited directly or indirectly over the device. The coating disposed over an implantable device can have a construct of any design. For instance, the coating can be a multi-layer structure that includes a primer layer (bottom), a reservoir layer and/or a topcoat or finishing coat layer (top).

Examples of polymers that can be used to coat an implantable device include poly(N-acetylglucosamine) (Chitin), Chitosan, poly(hydroxyvalerate), poly(lactide-co-glycolide), poly(hydroxybutyrate), poly(hydroxybutyrate-co-valerate), polyorthoester, polyanhydride, poly(L-lactic acid-co-caprolactone) (PLLA-CL), poly(D-lactic acid-co-caprolactone) (PDLA-CL), poly(DL-lactic acid-co-caprolactone) (PDLLA-CL), poly(D-lactic acid-glycolic acid (PDLA-GA), poly(L-lactic acid-glycolic acid (PLLA-GA), poly(DL-lactic acid-glycolic acid (PDLLA-GA), poly(L-lactic acid-co-caprolactone) (PLLA-CL), poly(D-lactic acid-co-caprolactone) (PDLA-CL), poly(DL-lactic acid-co-caprolactone) (PDLLA-CL), poly(glycolide-co-caprolactone) (PGA-CL), poly(thioesters), poly(trimethylene carbonate), polyethylene amide, polyethylene acrylate, poly(glycolic acid-co-trimethylene carbonate), co-poly(ether-esters) (e.g., PEO/PLA), polyphosphazenes, biomolecules (e.g., fibrin, fibrinogen, cellulose, starch, collagen and hyaluronic acid), polyurethanes, silicones, polyesters, polyolefins, polyisobutylene and ethylene-alphaolefin copolymers, acrylic polymers and copolymers other than polyacrylates, vinyl halide polymers and copolymers (e.g., polyvinyl chloride), polyvinyl ethers (e.g., polyvinyl methyl ether), polyvinylidene halides (e.g., polyvinylidene chloride), poly(vinylidene fluoride), poly(vinylidene fluoride-co-hexafluoropropylene), polyacrylonitrile, polyvinyl ketones, polyvinyl aromatics (e.g., polystyrene), polyvinyl esters (e.g., polyvinyl acetate), acrylonitrile-styrene copolymers, ABS resins, polyamides (e.g., Nylon 66 and polycaprolactam), polycarbonates, polyoxymethylenes, polyimides, polyethers, polyurethanes, rayon, rayon-triacetate, cellulose and derivates thereof (e.g., cellulose acetate, cellulose butyrate, cellulose acetate butyrate, cellophane, cellulose nitrate, cellulose propionate, cellulose ethers, and carboxymethyl cellulose), and copolymers thereof.

The implantable device may be delivered or introduced through a bodily lumen to a region, such as a lesion, in a vessel that requires treatment. Once it is placed in position it may be deployed. Deployment of a stent, for instance, corresponds to the expanding of the stent within the lumen at the treatment region. Delivery and deployment of a stent are accomplished by positioning the stent about one end of a catheter, inserting the end of the catheter through the skin into a bodily lumen, advancing the catheter in the bodily lumen to a desired treatment location, expanding the stent at the treatment location, and removing the catheter from the lumen. Other methods for delivering and using the implantable devices of the invention are well known to the skilled artisan.

### NO Deficiency Disorders

The compositions of the invention are useful in treating disorders resulting from NO deficiency or disorders that cause NO deficiency. Reasons for NO deficiency include 1) NOS dysfunction, resulting in the inability to produce NO from L-arginine in the blood vessels; 2) poor diet with insufficient nitrates and/or excess sugar intake; 3) oral dysbiosis or the inability of oral bacteria to convert dietary sources of nitrate into NO; 4) genetic disorder or weakness that affect NO production (e.g., endothelial dysfunction, argininosuccinic aciduria, Huntington's disease, sickle cell disease, hyperhomocystinemia, acute chest syndrome, muscular dystrophy, dyslipidemia, hypertensive disorders of pregnancy (e.g., pre-eclampsia), or senescence (e.g., Alzheimer's disease)); and 5) sedentary lifestyle.

The compositions of the invention are useful in improving learning and memory related to aging and protecting the skin from sun damage. NO deficiency plays a definite role in aging. Aging can cause > 50% loss in endothelial function. Further, a loss of 75% of endothelium derived nitric oxide is seen in 70-80 year old subjects compared to a younger population of subjects. Abnormal vasodilation in certain arteries also occurs with aging. Collectively, these findings illustrate that endothelial function declines progressively with age, as a consequence of declining nitric oxide levels in healthy subjects as well as subjects with existing diseases or disorders. Reduced availability of nitric oxide may increase risk of cardiovascular disease, sexual dysfunction and Alzheimer's Disease. Aging impairs the mechanism through which NO in the brain induces sleep. Reduced NO production and impaired endothelia function is observed in obstructive sleep apnea (OSA).

The compositions of the invention are also useful in relieving the symptoms of NO deficiency. Many symptoms of nitric oxide insufficiency occur with age: loss of energy, loss of memory, decline in sexual health and performance, and aches and pains that over time can manifest as specific disease.

### Cardiovascular Disease

Cardiovascular disease is a vascular endothelial cell dysfunction and certain symptoms begin, including as conventional or above the heart and vascular system-on, atherosclerosis, hypertension, gojihyeol, coronary heart disease (heart attack), cerebrovascular diseases (stroke, dementia), peripheral vascular disease, arrhythmia, heart failure, congestive heart disease, cardiac disease and for at least the name of the heart and blood vessels.

Some of the main factors of cardiovascular disease include, expression of genetic factors, lifestyle habits, and complications of diabetes Endothelial cell dysfunction (endothelial dysfunction) is found abnormal relaxation of the blood vessels in patients with hypertension in 1990 (Panza JA et al., New England Journal of Medicine, 323: 22-27, 1990) that since high blood pressure, arteriosclerosis, hyperlipidemia, diabetes, obesity is a very comprehensive primary function disorders that further adds to cardiovascular disease. (H. Brunner. Et al, J. Hypertens, 2005, 23: 233-246). As epithelial cells endothelial cells that line along the heart, blood vessels and the lymphatic cavities, the main function is to produce a vasodilator and vasoconstrictor nerve agents to adjust both the vascular tone and structure. Nitric oxide carries a variety of functions in the maintenance of vascular homeostasis, including the control of vascular tone, inhibition of thrombosis, inhibition of platelet aggregation, regulation of the expression of endothelial adhesion molecules.

Atherosclerosis is the mechanism by which arteries that supply the heart muscle with oxygen-rich blood become progressively narrowed with cholesterol-rich plaque. These plaques grow and harden over time, reducing blood flow, rupturing and causing a heart attack. Interventions to prop and hold open coronary arteries, such as angioplasty and metal stents, are tremendously common. One critical problem with these therapies is failure due to blood vessel remodeling, called neointimal hyperplasia, whereby smooth muscle cells grow through the stent, re-narrowing the vessel (i.e. restenosis). Drug-eluting stents (DES), coated with drugs to prevent cell growth, do reduce vessel restenosis by limiting the proliferation of smooth muscle cells; however, the drugs also inhibit the growth of endothelial cells that line naturally line blood vessels and prevent clot formation. Patients must remain on potent medications to prevent blood clots, predisposing them to life-threatening bleeding complications. NO is a small molecule shown to inhibit smooth muscle cell proliferation and promote the growth and migration of endothelial cells. Disclosed herein is a novel lipid molecule for NO delivery, and incorporated it into liposomes and nanoparticle-based therapeutics such that enable NO delivery from a next generation DES and/or intra-arterial injection to prevent restenosis.

The compositions of the invention are also useful in treating cardiovascular diseases. Nitric oxide delivery, via DES and/or intra-arterial injection, would improve patient survival and outcomes following vascular interventions (e.g. angioplasty) as well as preventing myocardial infarction-induced heart damage. The invention has tremendous value in improving the success rate of vascular interventions as well as reducing long-term damage caused by atherosclerotic plaque rupture.

As used herein cardiovascular diseases include arteriosclerosis, coronary heart disease, ischemia, endothelium dysfunction, in particular those dysfunctions affecting blood vessel elasticity, restenosis, thrombosis, angina, high blood pressure, cardiomyopathy, hypertensive heart disease, heart failure, cor pulmonale, cardiac dysrhythmias, endocarditis, inflammatory cardiomegaly, myocarditis, myocardial infarction, valvular heart disease, stroke and cerebrovascular disease, aortic valve stenosis, congestive heart failure, and peripheral arterial disease

In some embodiments, the compositions of the invention will restore and/or improve cardiovascular parameters to normal ranges in a subject diagnosed with or at risk of a cardiovascular disease. Normal ranges of cardiovascular parameters include, an end-diastolic volume (EDV) from about 65-240 mL, an end-systolic volume (ESV) from about 16-143m L, a stroke volume from about 55-100 mL, an ejection fraction from about 55-70%, a heart rate from about 60-100 bpm, and/or cardiac output of about 4.0-8.0 L/min.

### Inflammatory Disease

The compositions of the invention may also be used to treat inflammatory diseases. Examples of inflammatory diseases include, acne vulgaris, asthma, autoimmune diseases (e.g., acute disseminated encephalomyelitis (ADEM), Addison's disease, agammaglbulinemia, alopecia areata, amyotrophic lateral sclerosis, ankylosing spondylitis, antiphospholipid syndrome, antisynthetase syndrome, atopic allergy, atopic dermatitis, autoimmune aplastic anemia, autoimmune cardiomyopathy, autoimmune enteropathy, autoimmunehemolytic anemia, autoimmune hepatitis, autoimmune inner ear disease, autoimmune lymphoproliferative syndrome, autoimmune peripheral neuropathy, autoimmune pancreatitis, autoimmune polyendocrine syndrome, autoimmune progesterone dermatitis, autoimmune thrombocytopenic purpura, autoimmune urticaria, autoimmune uveitis, Balo concentric sclerosis, Behcet's disease, Berger's disease, Bickerstaff's encephalitis, Blau syndrome, bullous pemphigoid, Castleman's disease, celiac disease, Chagas disease, chronic inflammatory demyelinating polyneuropathy, chronic recurrent multifocal osteomyelitis, chronic obstructive pulmonary disease, Churg-Strauss syndrome, cicatricial pemphigoid, Cogan syndrome, cold agglutinin disease, complement component 2 deficiency, contact dermatitis, cranial arteritis, CREST syndrome, Crohn's disease, Cushing's syndrome, cutaneous leukocytoclastic vasculitis, Dego's disease, Dercum's disease, dermatitis herpetiformis, dermatomyositis, diabetes mellitus type 1, diffuse cutaneous systemic sclerosis, Dressler's syndrome, drug-induced lupus, discoid lupus erythematosus, eczema, endometriosis, enthesitis-related arthritis, eosinophilic fasciitis, eosinophilic gastroenteritis, epidermolysis bullosa acquisita, erythema nodosum, erythroblastosis fetalis, essential mixed cryoglobulinemia, Evan's syndrome, fibrodysplasia ossificans progressive, fibrosing alveolitis, gastritis, gastrointestinal pemphigoid, giant cell arteritis, glomerulonephritis, Goodpasture's syndrome, Grave's disease, Guillain-Barre syndrome, Hashimoto's encephalopathy, Hashimoto's thyroiditis, Henoch-Schonlein purpura, herpes gestationis, hidradenitis suppurativa, Hughes-Stovin syndrome, hypogammaglobulinemia, idiopathic inflammatory demyelinating diseases, idiopathic pulmonary fibrosis, idiopathic thrombocytopenic purpura, IgA nephropathy, inclusion body myositis, chronic inflammatory demyelinating polyneuropathy, interstitial cystitis, juvenile idiopathic arthritis, Kawasaki's disease, Lambert-Eaton myasthenic syndrome, leukocytoclastic vasculitis, lichen planus, lichen sclerosus, linear IgA disease, lupus erythematosus, Majeed syndrome, Meniere's disease, microscopic polyangiitis, mixed connective tissue disease, morphea, Mucha-Habermann disease, myasthenia gravis, myositis, narcolepsy, neuromyelitis optica, neuromyotonia, ocular cicatricial pemphigoid, opsoclonus myoclonus syndrome, Ord's thyroiditis, palindromic rheumatism, PANDAS, paraneoplastic cerebellar degeneration, paroxysmal nocturnal hemoglobinuria, Parry Romberg syndrome, Parsonage-Turner syndrome, pars planitis, pemphigus vulgaris, pernicious anaemia, perivenous encephalomyelitis, POEMS syndrome, polyarteritis nodosa, polymyalgia rheumatic, polymyositis, primary biliary cirrhosis, primary sclerosing cholangitis, progressive inflammatory neuropathy, psoriatic arthritis, pyoderma gangrenosum, pure red cell aplasia, Rasmussen's encephalitis, raynaud phenomenon, relapsing polychondritis, Reiter's syndrome, restless leg syndrome, retroperitoneal fibrosis, rheumatic fever, Schnitzler syndrome, scleritis, scleroderma, serum sickness, Sjogren's syndrome, spondyloarthropathy, stiff person syndrome, subacute bacterial endocarditis, Susac's syndrome, Sweet's syndrome, sympathetic ophthalmia, Takayasu's arteritis, temporal arteritis, thrombocytopenia, Tolosa-Hunt syndrome, transverse myelitis, ulcerative colitis, undifferentiated connective tissue disease, undifferentiated spondyloarthropathy, vitiligo, and Wegener's granulomatosis), celiac disease, chronic prostatitis, glomerulonephritis, hypersensitivities, inflammatory bowel diseases, pelvic inflammatory disease, reperfusion injury, rheumatoid arthritis, sarcoidosis, transplant rejection, vasculitis, interstitial cystitis, and osteoarthritis.

Other Pathological conditions associated with oxidative stress and/or an imbalance in redox homeostasis.

The compositions of the invention may be useful in treating other conditions associated with oxidative stress including autism, schizophrenia, bipolar disorder, fragile X syndrome, sickle cell disease, chronic fatigue syndrome, osteoarthritis cataract, macular degeneration, toxic hepatitis, viral hepatitis, cirrhosis, chronic hepatitis, oxidative stress from dialysis, renal toxicity, kidney failure, ulcerative colitis, bacterial infection, viral infections, such as HIV and AIDS, herpes, ear infection, upper respiratory tract diseases, hypertension, balding and hair loss, over-training syndrome related to athletic performance, eczema, scleroderma, atopic dermatitis, polymyositis, and dermatitis herpetiformis.

### Diabetes

Compositions of the invention may also be useful for treating diabetes and its complications. Diabetes can be any metabolic disease in which a person has high blood sugar, either because the body does not produce enough insulin, or because cells do not respond to the insulin that is produced. Examples of diabetes includes, type 1 diabetes mellitus, type 2 diabetes mellitus, gestational diabetes, congenital diabetes, cystic fibrosis-related diabetes, steroid diabetes, latent autoimmune diabetes of adults, and monogenic diabetes. Complications associated with diabetes include hypoglycemia, diabetic ketoacidosis, nonketotic hyperosmolar coma, cardiovascular disease, chronic renal failure, diabetic nephropathy, diabetic neuropathy, diabetes-related foot problems (e.g., diabetic foot ulcers), and diabetic retinopathy.

### Cancer

Other conditions that may be treated using compositions of the invention include cancers. Cancers are generally characterized by unregulated cell growth, formation of malignant tumors, and invasion to nearby parts of the body. Cancers may also spread to more distant parts of the body through the lymphatic system or bloodstream. Cancers may be a result of gene damage due to tobacco use, certain infections, radiation, lack of physical activity, obesity, and/or environmental pollutants. Cancers may also be a result of existing genetic faults within cells to cause diseases due to genetic heredity. Screenings may be used to detect cancers before any noticeable symptoms appear and treatment may be given to those who are at higher risks of developing cancers (e.g., people with a family history of cancers). Examples of screening techniques for cancer include physical examination, blood or urine tests, medical imaging, and/or genetic testing. Examples of cancers include: bladder cancer, breast cancer, colon and rectal cancer, endometrial cancer, kidney or renal cell cancer, leukemia, lung cancer, melanoma, Non-Hodgkin lymphoma, pancreatic cancer, prostate cancer, ovarian cancer, stomach cancer, wasting disease, and thyroid cancer.

The compositions containing an effective amount can be administered for prophylactic or therapeutic treatments. In prophylactic applications, compositions can be administered to a patient with a clinically determined predisposition or increased susceptibility to development of a NO deficiency disorder, cardiovascular diseases, hyperproliferative diseases (e.g., cancer), inflammatory diseases, diabetes, dyslipidemia, and other pathological conditions associated with oxidative stress, an imbalance in redox homeostasis, immune dysfunction, and/or endothelia dysfunction. Compositions of the invention can be administered to the patient (e.g., a human) in an amount sufficient to delay, reduce, or preferably prevent the onset of the clinical disease. In therapeutic applications, compositions are administered to a patient (e.g., a human) already suffering from a NO deficiency disorder, cardiovascular disease, hyperproliferative diseases (e.g., cancer), an inflammatory disease, diabetes, dyslipidemia, and other pathological conditions associated with oxidative stress, an imbalance in redox homeostasis, immune dysfunction, and/or endothelial dysfunction, in an amount sufficient to cure or at least partially arrest the symptoms of the condition and its complications. An amount adequate to accomplish this purpose is defined as a "therapeutically effective dose," an amount of a compound sufficient to substantially improve some symptom associated with a disease or a medical condition. For example, in the treatment of a NO deficiency disorder, cardiovascular disease, hyperproliferative diseases (e.g., cancer), an inflammatory disease, diabetes, dyslipidemia, and other pathological conditions associated with oxidative stress, an imbalance in redox homeostasis, immune dysfunction, and/or endothelia dysfunction, an agent or composition which decreases, prevents, delays, suppresses, or arrests any symptom of the disease or condition would be therapeutically effective. A therapeutically effective amount of an agent or composition is not required to cure a disease or condition but will provide a treatment for a disease or condition such that the onset of the disease or condition is delayed, hindered, or prevented, or the disease or condition symptoms are ameliorated, or the term of the disease or condition is changed or, for example, is less severe or recovery is accelerated in an individual.

Besides L-arginine and L-arginine hydrochloride, other examples of nitric oxide donors include D,L-arginine, D-arginine, or alkyl (e.g., ethyl, methyl, propyl, isopropyl, butyl, isobutyl, tert-butyl,.) esters of L-arginine and/or D-arginine (e.g., a methyl ester, an ethyl ester, a propyl ester, a butyl ester,) and/or salts thereof, as well as other derivatives of arginine and other nitric oxide donors. For instance, examples of pharmaceutically acceptable salts include hydrochloride, glutamate, butyrate, or glycolate (e.g., resulting in L-arginine glutamate, L-arginine butyrate, L-arginine glycolate, D-arginine hydrochloride, D-arginine glutamate,). Other examples of nitric oxide donors include L-arginine-based compounds such as, L-homoarginine, N-hydroxy-L-arginine, nitrosylated L-arginine, nitrosylated L-arginine, nitrosylated N-hydroxy-L-arginine, nitrosylated N-hydroxy-L-arginine, citrulline, omithine, linsidomine, nipride, glutamine, and salts thereof (e.g., hydrochloride, glutamate, butyrate, glycolate,). Still other examples of nitric oxide donors include S-nitrosothiols, nitrites, 2-hydroxy-2-nitrosohydrazines, or substrates of various forms of nitric oxide synthase. In some cases, the nitric oxide may be a compound that stimulates endogenous production of nitric oxide in vivo. Examples of such compounds include, L-arginine, substrates of various forms of nitric oxide synthase, certain cytokines, adenosine, bradykinin, calreticulin, bisacodyl, phenolphthalein, OH-arginine, or endothelein. It should be understood that, in any of the embodiments described herein that describe L-arginine, other nitric oxide donors may also be used instead, or in combination with, L-arginine, in other embodiments of the invention.

### Organ Transplantation

The compositions of the present invention may be useful to treat graft (e.g., organ, tissue) rejection. An organ transplant surgery replaces a failing organ with a healthy organ. Transplants may be the patient's own tissue (autografts; e.g., bone, bone marrow, and skin grafts); genetically identical (syngeneic or between monozygotic twins) donor tissue (isografts); genetically dissimilar donor tissue (allografts, or homografts); or, rarely, grafts from a different species (xenografts, or heterografts). Transplanted tissue may be cells (e.g., hematopoietic stem cell [HSC], lymphocyte, and pancreatic islet cell transplants,); parts or segments of an organ (e.g., hepatic or pulmonary lobar transplants and skin grafts,), entire organs (e.g., heart, lung, kidney, liver, pancreas, intestine, stomach, testis, hand transplants,), tissues (e.g., cornea, skin, islets of Langerhans, bone marrow, blood, blood vessels, heart valve, bone, composite tissue grafts,). Tissues may be grafted to an anatomically normal site (orthotopic; e.g., heart transplants) or abnormal site (heterotopic; e.g., a kidney transplanted into the iliac fossa). With rare exceptions, clinical transplantation uses allografts from living related, living unrelated, or deceased donors. Living donors are often used for kidney and HSC transplants and less frequently for segmental liver, pancreas, and lung transplants.

Organ and tissue transplantation is the preferred clinical approach to treat patients suffering from organ failure or complications arising from diseases of specific organs and tissues. However, transplant patients face a lifetime of immunosuppressive therapy and the risk of losing the new organ due to rejection. Although improvements have been made in the transplantation process, rejection remains the most common complication following transplantation and is the major source of morbidity and mortality. Transplant rejection occurs when the immune system of the recipient of a transplant attacks the transplanted organ or tissue. Rejection is an adaptive immune response and is mediated through both T lymphocyte-mediated and humoral immune (antibodies) mechanisms.

Donor organs are mostly stored in a cold environment for preservation (e.g., static cold preservation) because the metabolic rate of eukaryotic cells decline from two to three times at 10°C of reduction in the temperature in which they are. The technique requires the blood fast removal, fast organ cooling and a balance between the preservation solution and the organ. The preservation conditions are stressful and may cause damages resulting from ischemia (preservation hypothermic conditions) and reperfusion (transplantation in the donor). The preservation technique in hypothermic conditions has been applied first in 1952 by Lefevbre and Nizet, in France. Since then, only a few advances have been achieved in the organs preservation.

All allograft recipients are at risk of graft rejection; the recipient's immune system recognizes the graft as foreign and seeks to destroy it. Rejection of solid organs may be hyperacute, accelerated, acute, or chronic (late). These categories can be distinguished histopathologically and approximately by the time of onset. Recipients of grafts containing immune cells (particularly e.g., bone marrow, intestine, and liver) are at risk of graft-vs-host disease (GVHD). GVHD occurs when donor T cells react against recipient's self-antigens. It can include inflammatory damage to tissues, especially the liver, intestine, and skin, as well as blood dyscrasia (Information available from www.merckmanuals.com/professional/immunology-allergic-disorders/transplantation/ overview-of-transplantation). Organ rejection and/or GVHD may occur after heart, heart valve, lung, kidney, liver, pancreas, intestine, skin blood vessel, bone marrow, stem cell, bone, or islet cell transplantation. An islet cell transplantation can be performed to prevent the onset of diabetes or as a treatment of diabetes (Information available from U.S. Application Publication No. 2016/0311914).

Current methods to reduce the risk of these complications is minimized by pre-transplantation screening and immunosuppressive therapy during and after transplantation. The immunotherapy for solid organ transplantation is primarily T lymphocyte-directed and focused on preventing acute rejection. Immunosuppressants are primarily responsible for the success of transplantation. Treatment regimens include corticosteroids, calcineurin inhibitors (CNIs; e.g., cyclosporine, tacrolimus), cyclosporine, tacrolimusis, purine metabolism inhibitors (e.g., azathioprine and mycophenolate mofetil), rapamycins (e.g., sirolimus, everolimus), immunosuppressive immunoglobulins (e.g, antilymphocyte globulin [ALG], antithymocyte globulin [ATG]), monoclonal antibodies (mAbs; e.g., mAbs directed against T cells, OKT3, anti-IL-2 receptor monoclonal antibodies), irradiation. However, immunosuppressants suppress all immune responses and contribute to many posttransplantation complications, including development of cancer, acceleration of cardiovascular disease, and even death due to overwhelming infection. Allograft survival rates in the non-sensitized, cross-match negative recipient are quite good. However, long-term allograft survival rates remain unsatisfactory; which demonstrates that transplantation tolerance remains an unfulfilled goal. Thus, there remains a need for methods to promote organ or tissue transplantation tolerance in patients.

The immunosuppressive drugs currently used for the therapeutic treatment and handling of the organs rejection are focused on the inhibition of the alloreactive cell activation. Some severe side effects are hypertension, nephrotoxicity, central nervous system dysfunction (e.g., shivering, headache, depression, paresthesia, blurry vision), increased risk of viral, bacterial or fungal infections, increased risk of tumors occurrence, lack of appetite, nausea; some patients are resistant to the drugs and the combination of several drugs is necessary; high cost of the drugs; some drugs demonstrate adverse interactions with other drugs, such as antibiotics, non-steroidal anti-inflammatory, antiepileptic, antifungal and also immunization, such as German measles and polio.

Clinically ischemia/reperfusion injury is associated with delayed graft function, graft rejection, chronic rejection and chronic graft dysfunction (Salvadori et al. World J Transplant (2015) 5(2): 52-67).

Kidney transplantation is the most common type of solid organ transplantation. More than one half of donated kidneys come from previously healthy, brain-dead individuals. About one third of these kidneys are marginal, with physiologic or procedure-related damage, but are used because demand is so great. More kidneys from non-heart-beating donors (called donation-after-cardiac-death [DCD] grafts) are being used. The remaining donated kidneys (about another 40%) come from living donors; because of limited supply, allografts from carefully selected living unrelated donors are being increasingly used. Living donors relinquish reserve renal capacity, may put themselves at risk of procedural and long-term morbidity, and may have psychologic conflicts about donation; therefore, they are evaluated for normal bilateral renal function, absence of systemic disease, histocompatibility, emotional stability, and ability to give informed consent.

The donor kidney is removed during a laparoscopic (or rarely, an open) procedure, perfused with cooling solutions containing relatively large concentrations of poorly permeating substances (eg, mannitol, hetastarch) and electrolyte concentrations approximating intracellular levels, then stored in an iced solution. Kidneys preserved this way usually function well if transplanted within 24 h. Although not commonly used, continuous pulsatile hypothermic perfusion with an oxygenated, plasma-based perfusate can extend ex vivo viability up to 48 h.

Immunosuppressive regimens vary. Commonly, calcineurin inhibitors are begun immediately after transplantation in doses titrated to minimize toxicity and rejection while maintaining trough blood levels high enough to prevent rejection. On the day of transplantation, IV or oral corticosteroids are also given; dose is tapered over the following weeks depending on the protocol used. Despite use of immunosuppressants, about 20% of kidney transplant recipients have one or more rejection episodes within the first year after transplantation. Most episodes are easily treated with a corticosteroid bolus; however, they contribute to long-term insufficiency, graft failure, or both. Signs of rejection vary by type of rejection. Chronic allograft nephropathy refers to graft insufficiency or failure ≥ 3 mo after transplantation. Most rejection episodes and other complications occur within 3 to 4 mo after transplantation; most patients then return to more normal health and activity but must take maintenance doses of immunosuppressants indefinitely.

At 1 yr after kidney transplantation, survival rates are in living-donor grafts: 98% (patients) and 94% (grafts); deceased-donor grafts: 95% (patients) and 88% (grafts); subsequent annual graft loss rates are 3 to 5% with a living-donor graft and 5 to 8% with a deceased-donor graft. Among patients whose graft survives the first year, half die of other causes with the graft functioning normally; half develop chronic allograft nephropathy with the graft malfunctioning in 1 to 5 yr.

In a specific patient, the most recently obtained creatinine levels should be compared with previous levels; a sudden increase in creatinine indicates the need to consider rejection or another problem (e.g., vascular compromise, obstruction of the ureter). Ideally, serum creatinine should be normal in all posttransplant patients 4 to 6 wk after kidney transplantation (Information available from the Merck Manual: www.merckmanuals.com/professional/immunology-allergic-disorders/transplantation/ kidney-transplantation).

The invention, in some embodiments, provides nanostructures that deliver NO-releasing phospholipids and/or NO-releasing liposomes and/or HDL-NPs and/or NO releasing HDL-NPs via drug eluting stents and/or intra-arterial injection NO to a cell to prevent or decrease the rejection of transplanted organs. According to some aspects, the nanostructure reduces the risk of rejection of the donor organ relative to the risk of a donor organ transplanted without exposure to the nanostructure.

NO plays a pivotal role in regulating vessel wall homeostasis and as such it is an important component of the vascular system.

The vascular system is made up of the vessels that carry blood and lymph through the body. The arteries and veins carry blood throughout the body, delivering oxygen and nutrients to the body tissues and taking away tissue waste matter. The lymph vessels carry lymphatic fluid (a clear, colorless fluid containing water and blood cells). The lymphatic system helps to protect and maintain the fluid environment of the body by filtering and draining lymph away from each region of the body. The vessels of the blood circulatory system are:
(1) Arteries. Blood vessels that carry oxygenated blood away from the heart to the body.
(2) Veins. Blood vessels that carry blood from the body back into the heart.
(3) Capillaries. Tiny blood vessels between arteries and veins that distribute oxygen-rich blood to the body.

Blood moves through the circulatory system as a result of being pumped out by the heart. Blood leaving the heart through the arteries is saturated with oxygen. The arteries break down into smaller and smaller branches in order to bring oxygen and other nutrients to the cells of the body's tissues and organs. As blood moves through the capillaries, the oxygen and other nutrients move out into the cells, and waste matter from the cells moves into the capillaries. As the blood leaves the capillaries, it moves through the veins, which become larger and larger to carry the blood back to the heart.

In addition to circulating blood and lymph throughout the body, the vascular system functions as an important component of other body systems. Examples include:
(1) Respiratory system. As blood flows through the capillaries in the lungs, carbon dioxide is given up and oxygen is picked up. The carbon dioxide is expelled from the body through the lungs, and the oxygen is taken to the body tissues by the blood.
(2) Digestive system. As food is digested, blood flows through the intestinal capillaries and picks up nutrients, such as glucose (sugar), vitamins, and minerals. These nutrients are delivered to the body tissues by the blood.
(3) Kidneys and urinary system. Waste materials from the body tissues are filtered out from the blood as it flows through the kidneys. The waste material then leaves the body in the form of urine.
(4) Temperature control. Regulation of the body's temperature is assisted by the flow of blood among the different parts of the body. Heat is produced by the body's tissues as they go through the processes of breaking down nutrients for energy, making new tissue, and giving up waste matter.

A vascular disease is a condition that affects the arteries and/or veins. Most often, vascular disease affects blood flow, either by blocking or weakening blood vessels, or by damaging the valves that are found in veins. Organs and other body structures may be damaged by vascular disease as a result of decreased or completely blocked blood flow.

Causes of vascular disease include, but are not limited to:
(1) Atherosclerosis. Atherosclerosis (a buildup of plaque, which is a deposit of fatty substances, cholesterol, cellular waste products, calcium, and fibrin in the inner lining of an artery) is the most common cause of vascular disease. It is unknown exactly how atherosclerosis begins or what causes it. Atherosclerosis is a slow, progressive, vascular disease that may start as early as childhood. However, the disease has the potential to progress rapidly. It is generally characterized by the accumulation of fatty deposits along the innermost layer of the arteries. If the disease process progresses, plaque formation may take place. This thickening narrows the arteries and can decrease blood flow or completely block the flow of blood to organs and other body tissues and structures.
(2) Embolus/thrombus. A blood vessel may be blocked by an embolus (a tiny mass of debris that moves through the bloodstream) or a thrombus (a blood clot).
(3) Inflammation. In general, inflammation of blood vessels is referred to as vasculitis, which includes a range of disorders. Inflammation may lead to narrowing and/or blockage of blood vessels.
(4) Traumalinjury. Trauma or injury involving the blood vessels may lead to inflammation or infection, which can damage the blood vessels and lead to narrowing and/or blockage.

Because the functions of the blood vessels include supplying all organs and tissues of the body with oxygen and nutrients, removal of waste products, fluid balance, and other functions, conditions that affect the vascular system may affect the part(s) of the body supplied by a particular vascular network, such as the coronary arteries of the heart.

As a free radical gas, NO has an extremely short half-life. In certain instances, it may be desirable to increase the effective amount of NO in a cell, tissue, or organ in order to induce vascular relaxation, vascular dilation, vascularization, oxygenation, or other NO mediated biological process. The compositions and formulations of the present invention may be used in combination with either conventional methods of treatment or therapy or may be used separately from conventional methods of treatment or therapy. When the compositions and formulations of this invention are administered in combination therapies with other agents, they may be administered sequentially or concurrently to an individual. Alternatively, pharmaceutical compositions according to the present invention include a combination of a nitric oxide releasing HDL-NP of the present invention optionally in association with a pharmaceutically acceptable excipient, as described herein, and another therapeutic or prophylactic agent known in the art. The nanoparticles are believed to act via a specific receptor-mediated pathway, such as the SR-B1 receptor, limiting effects to those cells expressing the receptor.

As described herein, a "reservoir molecule" refers to a molecule with the ability to complex with NO. For instance, the reservoir molecule may be a lipid having an NO donating group. The reservoir molecule (e.g., nitrosylated lipid) is able to release a NO group at a target site. In some embodiments, the reservoir molecule is a lipid molecule that has been modified to contain a NO-donating group. An example of a modified lipid is an S-nitrosylated lipid or N-nitrosylated lipid. In some embodiments, the reservoir molecule is a lipid molecule that has been modified to include other molecules that can donate an NO group. Examples include diazeniumdiolates (also known as NONOates) (See e.g., Ramamurthi et al. (1997) Chem Res Toxicol 10(4):408-413). Diazeniumdiolates typically have half-lives of milliseconds in biological systems (e.g., cell culture media, plasma, etc.). The reservoir molecule (e.g., nitrosylated lipid) is able to release a NO group at a target site. In other embodiments, the reservoir molecules (e.g., heads of phospholipids) can be modified to include a wide range of moieties, including but not limited to fluorophores, MR contrast agents , be biotinylated or be glycosylated.

In other embodiments, the reservoir molecule is not a lipid. An example of non-lipid reservoir molecules, includes but is not limited to, glutathione (See e.g., Pompella et al., Biochem Pharmacol (2003) 66(8):1499-1503). Glutathione is a tripeptide that acts as a natural NO reservoir in vivo. In some embodiments, the structure, nanostructure or nanoparticle (e.g., HDL nanoparticle) described herein contains one or more glutathiones. In some embodiments, the free thiol in glutathione is modified (e.g., S-nitrosylated).

In some aspects, the invention is a nanostructure composed of a nanostructure core of an inorganic material surrounded by a shell of a lipid layer (e.g., lipid shell). The nanostructure may also include a protein such as an apolipoprotein.

The shell may have an inner surface and an outer surface, such that the therapeutic agent and/or the apolipoprotein may be adsorbed on the outer shell and/or incorporated between the inner surface and outer surface of the shell.

The shell may also have a therapeutic profile for a therapeutic agent. A "therapeutic profile" as used herein refers to a composition of lipids and/or proteins that promote binding of a particular therapeutic agent. Each therapeutic agent has a particular shape, charge, and degree or level of hydrophobicity that may contribute to its ability to bind to the shell and or protein bound to the surface. The binding capacity as well as binding affinity between the therapeutic agent and the nanostructure may be regulated by modification to the therapeutic profile. For instance, a particular combination of lipids may provide an optimal surface for binding to a small molecule or protein. Positively charged head groups in the outer layer are shown to decrease the binding affinity, while negatively charged lipid head groups increase the binding affinity.

Examples of nanostructures that can be used in the methods are described herein are now described. The structure (e.g., a synthetic structure or synthetic nanostructure) has a core and a shell surrounding the core. In embodiments in which the core is a nanostructure, the core includes a surface to which one or more components can be optionally attached. For instance, in some cases, core is a nanostructure surrounded by shell, which includes an inner surface and an outer surface. The shell may be formed, at least in part, of one or more components, such as a plurality of lipids, which may optionally associate with one another and/or with surface of the core. For example, components may be associated with the core by being covalently attached to the core, physiosorbed, chemisorbed, or attached to the core through ionic interactions, hydrophobic and/or hydrophilic interactions, electrostatic interactions, van der Waals interactions, or combinations thereof. In one particular embodiment, the core includes a gold nanostructure and the shell is attached to the core through a gold-thiol bond.

A number of therapeutic agents are typically associated with the shell of a nanostructure. For instance, at least 20 therapeutic agents may be associated per structure. In general at least 20-30, 20-40, 20-50, 25-30, 25-40, 25-50, 30-40, 30-50, 35-40, 35-50, 40-45, 40-50, 45-50, 50-100 or 30-100 therapeutic agents may be associated per structure.

Optionally, components can be crosslinked to one another. Crosslinking of components of a shell can, for example, allow the control of transport of species into the shell, or between an area exterior to the shell and an area interior of the shell. For example, relatively high amounts of crosslinking may allow certain small, but not large, molecules to pass into or through the shell, whereas relatively low or no crosslinking can allow larger molecules to pass into or through the shell. Additionally, the components forming the shell may be in the form of a monolayer or a multilayer, which can also facilitate or impede the transport or sequestering of molecules. In one exemplary embodiment, shell includes a lipid bilayer that is arranged to sequester cholesterol and/or control cholesterol efflux out of cells, as described herein.

It should be understood that a shell which surrounds a core need not completely surround the core, although such embodiments may be possible. For example, the shell may surround at least 50%, at least 60%, at least 70%, at least 80%, at least 90%, or at least 99% of the surface area of a core. In some cases, the shell substantially surrounds a core. In other cases, the shell completely surrounds a core. The components of the shell may be distributed evenly across a surface of the core in some cases, and unevenly in other cases. For example, the shell may include portions (e.g., holes) that do not include any material in some cases. If desired, the shell may be designed to allow penetration and/or transport of certain molecules and components into or out of the shell, but may prevent penetration and/or transport of other molecules and components into or out of the shell. The ability of certain molecules to penetrate and/or be transported into and/or across a shell may depend on, for example, the packing density of the components forming the shell and the chemical and physical properties of the components forming the shell. The shell may include one layer of material, or multilayers of materials in some embodiments.

The core of the nanostructure whether being a nanostructure core or a hollow core, may have any suitable shape and/or size. For instance, the core may be substantially spherical, non-spherical, oval, rod-shaped, pyramidal, cube-like, disk-shaped, wire-like, or irregularly shaped. The core (e.g., a nanostructure core or a hollow core) may have a largest cross-sectional dimension (or, sometimes, a smallest cross-section dimension) of, for example, less than or equal to about 500 nm, less than or equal to about 250 nm, less than or equal to about 100 nm, less than or equal to about 75 nm, less than or equal to about 50 nm, less than or equal to about 40 nm, less than or equal to about 35 nm, less than or equal to about 30 nm, less than or equal to about 25 nm, less than or equal to about 20 nm, less than or equal to about 15 nm, or less than or equal to about 5 nm. In some cases, the core has an aspect ratio of greater than about 1:1, greater than 3:1, or greater than 5:1. As used herein, "aspect ratio" refers to the ratio of a length to a width, where length and width measured perpendicular to one another, and the length refers to the longest linearly measured dimension.

The core may be formed of an inorganic material. The inorganic material may include, for example, a metal (e.g., Ag, Au, Pt, Fe, Cr, Co, Ni, Cu, Zn, and other transition metals), a semiconductor (e.g., silicon, silicon compounds and alloys, cadmium selenide, cadmium sulfide, indium arsenide, and indium phosphide), or an insulator (e.g., ceramics such as silicon oxide). The inorganic material may be present in the core in any suitable amount, e.g., at least 1 wt%, 5 wt%, 10 wt%, 25 wt%, 50 wt%, 75 wt%, 90 wt%, or 99 wt%. In one embodiment, the core is formed of 100 wt% inorganic material. The nanostructure core may, in some cases, be in the form of a quantum dot, a carbon nanotube, a carbon nanowire, or a carbon nanorod. In some cases, the nanostructure core comprises, or is formed of, a material that is not of biological origin. In some embodiments, a nanostructure includes or may be formed of one or more organic materials such as a synthetic polymer and/or a natural polymer. Examples of synthetic polymers include non-degradable polymers such as polymethacrylate and degradable polymers such as polylactic acid, polyglycolic acid and copolymers thereof. Examples of natural polymers include hyaluronic acid, chitosan, and collagen.

Furthermore, a shell of a structure can have any suitable thickness. For example, the thickness of a shell may be at least 10 Angstroms, at least 0.1 nm, at least 1 nm, at least 2 nm, at least 5 nm, at least 7 nm, at least 10 nm, at least 15 nm, at least 20 nm, at least 30 nm, at least 50 nm, at least 100 nm, or at least 200 nm (e.g., from the inner surface to the outer surface of the shell). In some cases, the thickness of a shell is less than 200 nm, less than 100 nm, less than 50 nm, less than 30 nm, less than 20 nm, less than 15 nm, less than 10 nm, less than 7 nm, less than 5 nm, less than 3 nm, less than 2 nm, or less than 1 nm (e.g., from the inner surface to the outer surface of the shell). Such thicknesses may be determined prior to or after sequestration of molecules as described herein.

The shell of a structure described herein may comprise any suitable material, such as a hydrophobic material, a hydrophilic material, and/or an amphiphilic material. Although the shell may include one or more inorganic materials such as those listed above for the nanostructure core, in many embodiments the shell includes an organic material such as a lipid or certain polymers. In one set of embodiments, a structure described herein or a portion thereof, such as a shell of a structure, includes one or more natural or synthetic lipids or lipid analogs (i.e., lipophilic molecules). One or more lipids and/or lipid analogues may form a single layer or a multi-layer (e.g., a bilayer) of a structure. In some instances where multi-layers are formed, the natural or synthetic lipids or lipid analogs interdigitate (e.g., between different layers). Examples of natural or synthetic lipids or lipid analogs include fatty acyls, glycerolipids, glycerophospholipids, sphingolipids, saccharolipids and polyketides (derived from condensation of ketoacyl subunits), and sterol lipids and prenol lipids (derived from condensation of isoprene subunits).

In one particular set of embodiments, a structure described herein includes one or more phospholipids. The one or more phospholipids may include, for example, 1,2-Dipalmitoyl-sn-Glycero-3-Phosphothioethanol, phosphatidylcholine, phosphatidylglycerol, lecithin, β, γ-dipalmitoyl-α-lecithin, sphingomyelin, phosphatidylserine, phosphatidic acid, N-(2,3-di(9-(Z)-octadecenyloxy))-prop-1-yl-N,N,N-trimethylammonium chloride, phosphatidylethanolamine, lysolecithin, lysophosphatidylethanolamine, phosphatidylinositol, cephalin, cardiolipin, cerebrosides, dicetylphosphate, dioleoylphosphatidylcholine, dipalmitoylphosphatidylcholine, dipalmitoylphosphatidylglycerol, dioleoylphosphatidylglycerol, palmitoyl-oleoyl-phosphatidylcholine, di-stearoyl-phosphatidylcholine, stearoyl-palmitoyl-phosphatidylcholine, di-palmitoyl-phosphatidylethanolamine, di-stearoyl-phosphatidylethanolamine, di-myrstoyl-phosphatidylserine, di-oleyl-phosphatidylcholine, 1,2-dipalmitoyl-sn-glycero-3-phosphothioethanol, and combinations thereof. In some cases, a shell (e.g., a bilayer) of a structure includes 50-200 natural or synthetic lipids or lipid analogs (e.g., phospholipids). For example, the shell may include less than about 500, less than about 400, less than about 300, less than about 200, or less than about 100 natural or synthetic lipids or lipid analogs (e.g., phospholipids), e.g., depending on the size of the structure.

Non-phosphorus containing lipids may also be used such as stearylamine, docecylamine, acetyl palmitate, and fatty acid amides. In other embodiments, other lipids such as fats, oils, waxes, cholesterol, sterols, fat-soluble vitamins (e.g., vitamins A, D, E and K), glycerides (e.g., monoglycerides, diglycerides, triglycerides) can be used to form portions of a structure described herein.

A portion of a structure described herein such as a shell or a surface of a nanostructure may optionally include one or more alkyl groups, e.g., an alkane-, alkene-, or alkyne-containing species, that optionally imparts hydrophobicity to the structure. An "alkyl" group refers to a saturated aliphatic group, including a straight-chain alkyl group, branched-chain alkyl group, cycloalkyl (alicyclic) group, alkyl substituted cycloalkyl group, and cycloalkyl substituted alkyl group. The alkyl group may have various carbon numbers, e.g., between C2 and C40, and in some embodiments may be greater than C5, C10, C15, C20, C25, C30, or C35. In some embodiments, a straight chain or branched chain alkyl may have 30 or fewer carbon atoms in its backbone, and, in some cases, 20 or fewer. In some embodiments, a straight chain or branched chain alkyl may have 12 or fewer carbon atoms in its backbone (e.g., C1-C12 for straight chain, C3-C12 for branched chain), 6 or fewer, or 4 or fewer. Likewise, cycloalkyls may have from 3-10 carbon atoms in their ring structure, or 5, 6 or 7 carbons in the ring structure. Examples of alkyl groups include, but are not limited to, methyl, ethyl, propyl, isopropyl, cyclopropyl, butyl, isobutyl, tert-butyl, cyclobutyl, hexyl, and cyclochexyl.

The alkyl group may include any suitable end group, e.g., a thiol group, an amino group (e.g., an unsubstituted or substituted amine), an amide group, an imine group, a carboxyl group, or a sulfate group, which may, for example, allow attachment of a ligand to a nanostructure core directly or via a linker. For example, where inert metals are used to form a nanostructure core, the alkyl species may include a thiol group to form a metal-thiol bond. In some instances, the alkyl species includes at least a second end group. For example, the species may be bound to a hydrophilic moiety such as polyethylene glycol. In other embodiments, the second end group may be a reactive group that can covalently attach to another functional group. In some instances, the second end group can participate in a ligand/receptor interaction (e.g., biotin/streptavidin).

In some embodiments, the shell includes a polymer. For example, an amphiphilic polymer may be used. The polymer may be a diblock copolymer, a triblock copolymer, e.g., where one block is a hydrophobic polymer and another block is a hydrophilic polymer. For example, the polymer may be a copolymer of an α-hydroxy acid (e.g., lactic acid) and polyethylene glycol. In some cases, a shell includes a hydrophobic polymer, such as polymers that may include certain acrylics, amides and imides, carbonates, dienes, esters, ethers, fluorocarbons, olefins, sytrenes, vinyl acetals, vinyl and vinylidene chlorides, vinyl esters, vinyl ethers and ketones, and vinylpyridine and vinylpyrrolidones polymers. In other cases, a shell includes a hydrophilic polymer, such as polymers including certain acrylics, amines, ethers, styrenes, vinyl acids, and vinyl alcohols. The polymer may be charged or uncharged. As noted herein, the particular components of the shell can be chosen so as to impart certain functionality to the structures.

Where a shell includes an amphiphilic material, the material can be arranged in any suitable manner with respect to the nanostructure core and/or with each other. For instance, the amphiphilic material may include a hydrophilic group that points towards the core and a hydrophobic group that extends away from the core, or, the amphiphilic material may include a hydrophobic group that points towards the core and a hydrophilic group that extends away from the core. Bilayers of each configuration can also be formed.

The structures described herein may also include one or more proteins, polypeptides and/or peptides (e.g., synthetic peptides, amphiphilic peptides). In one set of embodiments, the structures include proteins, polypeptides and/or peptides that can increase the rate of cholesterol transfer or the cholesterol-carrying capacity of the structures. The one or more proteins or peptides may be associated with the core (e.g., a surface of the core or embedded in the core), the shell (e.g., an inner and/or outer surface of the shell, and/or embedded in the shell), or both. Associations may include covalent or non-covalent interactions (e.g., hydrophobic and/or hydrophilic interactions, electrostatic interactions, van der Waals interactions).

An example of a suitable protein that may associate with a structure described herein is an apolipoprotein, such as apolipoprotein A (e.g., apo A-I, apo A-II, apo A-IV, and apo A-V), apolipoprotein B (e.g., apo B48 and apo B 100), apolipoprotein C (e.g., apo C-I, apo C-II, apo C-III, and apo C-IV), and apolipoproteins D, E, and H. Specifically, apo A1 , apo A2 , and apo E promote transfer of cholesterol and cholesteryl esters to the liver for metabolism and may be useful to include in structures described herein. Additionally or alternatively, a structure described herein may include one or more peptide analogues of an apolipoprotein, such as one described above. A structure may include any suitable number of, e.g., at least 1, 2, 3, 4, 5, 6, or 10, apolipoproteins or analogues thereof. In certain embodiments, a structure includes 1-6 apolipoproteins, similar to a naturally occurring HDL particle. Of course, other proteins (e.g., non-apolipoproteins) can also be included in structures described herein.

Optionally, one or more enzymes may also be associated with a structure described herein. For example, lecithin-cholesterol acyltransferase is an enzyme which converts free cholesterol into cholesteryl ester (a more hydrophobic form of cholesterol). In naturally-occurring lipoproteins (e.g., HDL and LDL), cholesteryl ester is sequestered into the core of the lipoprotein, and causes the lipoprotein to change from a disk shape to a spherical shape. Thus, structures described herein may include lecithin-cholesterol acyltransferase to mimic HDL and LDL structures. Other enzymes such as cholesteryl ester transfer protein (CETP) which transfers esterified cholesterol from HDL to LDL species may also be included.

It should be understood that the components described herein, such as the lipids, phospholipids, alkyl groups, polymers, proteins, polypeptides, peptides, enzymes, bioactive agents, nucleic acids, and species for targeting described above (which may be optional), may be associated with a structure in any suitable manner and with any suitable portion of the structure, e.g., the core, the shell, or both. For example, one or more such components may be associated with a surface of a core, an interior of a core, an inner surface of a shell, an outer surface of a shell, and/or embedded in a shell.

Additionally, the components described herein, such as the lipids, phospholipids, alkyl groups, polymers, proteins, polypeptides, peptides, enzymes, bioactive agents, nucleic acids, and species for targeting described above, may be associated with a structure described herein prior to administration to a subject or biological sample and/or after administration to a subject or biological sample. For example, in some cases a structure described herein includes a core and a shell which is administered in vivo or in vitro, and the structure has a greater therapeutic effect after sequestering one or more components (e.g., an apolipoprotein) from a subject or biological sample. That is, the structure may use natural components from the subject or biological sample to increase efficacy of the structure after it has been administered.

A variety of methods can be used to fabricate the nanostructures described herein. Examples of methods are provided in International Patent Publication No. WO/2009/131704, filed April 24, 2009 and entitled, "Nanostructures Suitable for Sequestering Cholesterol and Other Molecules".

As described herein, the inventive structures may be used in "pharmaceutical compositions" or "pharmaceutically acceptable" compositions, which comprise a therapeutically effective amount of one or more of the structures described herein, formulated together with one or more pharmaceutically acceptable carriers, additives, and/or diluents. The pharmaceutical compositions described herein may be useful for treating cancer, vascular diseases, angiogenesis, ischemia-reperfusion (e.g., ischemia reperfusion injury following organ transplantation), prevention of restenosis following vascular interventions (e.g. angioplasty), ameliorate endothelial dysfunction and artery stiffening that occur in atherosclerosis, as a blood pressure therapy, reduce ischemia-reperfusion injury following myocardial infarction, reduce atherosclerotic plaque burden or other conditions. It should be understood that any suitable structures described herein can be used in such pharmaceutical compositions, including those described in connection with the figures. In some cases, the structures in a pharmaceutical composition have a nanostructure core comprising an inorganic material and a shell substantially surrounding and attached to the nanostructure core.

The pharmaceutical compositions may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream or foam; sublingually; ocularly; transdermally; or nasally, pulmonary and to other mucosal surfaces.

The phrase "pharmaceutically acceptable" is employed herein to refer to those structures, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: water soluble antioxidants, such as ascorbic acid, cysteine hydrochloride, sodium bisulfate, sodium metabisulfite, and sodium sulfite; oil-soluble antioxidants, such as ascorbyl palmitate, butylated and hydroxyanisole (BHA), butylated hydroxytoluene (BHT), lecithin, propyl gallate, alpha-tocopherol; and metal chelating agents, such as citric acid, ethylenediamine tetraacetic acid (EDTA), sorbitol, tartaric acid, and phosphoric acid.

The structures described herein may be orally administered, parenterally administered, subcutaneously administered, and/or intravenously administered. In certain embodiments, a structure or pharmaceutical preparation is administered orally. In other embodiments, the structure or pharmaceutical preparation is administered intravenously. Alternative routes of administration include sublingual, intramuscular, and transdermal administrations.

Pharmaceutical compositions described herein include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, and the particular mode of administration. The amount of active ingredient that can be combined with a carrier material to produce a single dosage form will generally be that amount of the compound which produces a therapeutic effect. Generally, this amount will range from about 1% to about 99% of active ingredient, from about 5% to about 70%, or from about 10% to about 30%.

The inventive compositions suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes, each containing a predetermined amount of a structure described herein as an active ingredient. An inventive structure may also be administered as a bolus, electuary or paste.

In solid dosage forms of the invention for oral administration (capsules, tablets, pills, dragees, powders, and granules), the active ingredient is mixed with one or more pharmaceutically-acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol, glycerol monostearate, and non-ionic surfactants; absorbents, such as kaolin and bentonite clay; lubricants, such as talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made in a suitable machine in which a mixture of the powdered structure is moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms for oral administration of the structures described herein include pharmaceutically acceptable emulsions, microemulsions, solutions, dispersions, suspensions, syrups and elixirs. In addition to the inventive structures, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions described herein (e.g., for rectal or vaginal administration) may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the body and release the structures.

Dosage forms for the topical or transdermal administration of a structure described herein include powders, sprays, ointments, pastes, foams, creams, lotions, gels, solutions, patches and inhalants. The active compound may be mixed under sterile conditions with a pharmaceutically-acceptable carrier, and with any preservatives, buffers, or propellants which may be required.

The ointments, pastes, creams and gels may contain, in addition to the inventive structures, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the structures described herein, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a structure described herein to the body. Dissolving or dispersing the structure in the proper medium can make such dosage forms. Absorption enhancers can also be used to increase the flux of the structure across the skin. Either providing a rate controlling membrane or dispersing the structure in a polymer matrix or gel can control the rate of such flux.

Ophthalmic formulations, eye ointments, powders, and solutions are also contemplated as being within the scope of this invention.

Pharmaceutical compositions described herein suitable for parenteral administration comprise one or more inventive structures in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers, which may be employed in the pharmaceutical compositions described herein include water, ethanol, polyols (such as glycerol, propylene glycol, and polyethylene glycol), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the inventive structures may be facilitated by the inclusion of various antibacterial and antifungal agents, for example, paraben, chlorobutanol, phenol and sorbic acid. It may also be desirable to include isotonic agents, such as sugars, and sodium chloride into the compositions. In addition, prolonged absorption of the injectable pharmaceutical form may be brought about by the inclusion of agents which delay absorption such as aluminum monostearate and gelatin.

Delivery systems suitable for use with structures and compositions described herein include time-release, delayed release, sustained release, or controlled release delivery systems, as described herein. Such systems may avoid repeated administrations of the structures in many cases, increasing convenience to the subject and the physician. Many types of release delivery systems are available and known to those of ordinary skill in the art. They include, for example, polymer based systems such as polylactic and/or polyglycolic acid, polyanhydrides, and polycaprolactone; nonpolymer systems that are lipid-based including sterols such as cholesterol, cholesterol esters, and fatty acids or neutral fats such as mono-, di- and triglycerides; hydrogel release systems; silastic systems; peptide based systems; wax coatings; compressed tablets using conventional binders and excipients; or partially fused implants. Specific examples include, but are not limited to, erosional systems in which the composition is contained in a form within a matrix, or diffusional systems in which an active component controls the release rate. The compositions may be as, for example, microspheres, hydrogels, polymeric reservoirs, cholesterol matrices, or polymeric systems. In some embodiments, the system may allow sustained or controlled release of the active compound to occur, for example, through control of the diffusion or erosion/degradation rate of the formulation. In addition, a pump-based hardware delivery system may be used in some embodiments. The structures and compositions described herein can also be combined (e.g., contained) with delivery devices such as syringes, pads, patches, tubes, films, MEMS-based devices, and implantable devices.

Use of a long-term release implant may be particularly suitable in some cases. "Long-term release," as used herein, means that the implant is constructed and arranged to deliver therapeutic levels of the composition for at least about 30 or about 45 days, for at least about 60 or about 90 days, or even longer in some cases. Long-term release implants are well known to those of ordinary skill in the art, and include some of the release systems described above.

Injectable depot forms can be made by forming microencapsule matrices of the structures described herein in biodegradable polymers such as polylactide-polyglycolide. Depending on the ratio of structure to polymer, and the nature of the particular polymer employed, the rate of release of the structure can be controlled. Examples of other biodegradable polymers include poly(orthoesters) and poly(anhydrides).

When the structures described herein are administered as pharmaceuticals, to humans and animals, they can be given per se or as a pharmaceutical composition containing, for example, about 0.1% to about 99.5%, or about 0.5% to about 90% of structures in combination with a pharmaceutically acceptable carrier.

The administration may be localized (e.g., to a particular region, physiological system, tissue, organ, or cell type) or systemic, depending on the condition to be treated. For example, the composition may be administered through parental injection, implantation, orally, vaginally, rectally, buccally, pulmonary, topically, nasally, transdermally, surgical administration, or any other method of administration where access to the target by the composition is achieved. Examples of parental modalities that can be used with the invention include intravenous, intradermal, subcutaneous, intracavity, intramuscular, intraperitoneal, epidural, or intrathecal. Examples of implantation modalities include any implantable or injectable drug delivery system. Oral administration may be useful for some treatments because of the convenience to the patient as well as the dosing schedule.

Regardless of the route of administration selected, the structures described herein, which may be used in a suitable hydrated form, and/or the inventive pharmaceutical compositions, are formulated into pharmaceutically-acceptable dosage forms by conventional methods known to those of skill in the art.

The compositions described herein may be given in dosages, e.g., at the maximum amount while avoiding or minimizing any potentially detrimental side effects. The compositions can be administered in effective amounts, alone or in a combinations with other compounds. For example, when treating cancer, a composition may include the structures described herein and a cocktail of other compounds that can be used to treat cancer. When treating conditions associated with abnormal lipid levels, a composition may include the structures described herein and other compounds that can be used to reduce lipid levels (e.g., cholesterol lowering agents).

The phrase "therapeutically effective amount" as used herein means that amount of a material or composition comprising an inventive structure which is effective for producing some desired therapeutic effect in a subject at a reasonable benefit/risk ratio applicable to any medical treatment. Accordingly, a therapeutically effective amount may, for example, prevent, minimize, or reverse disease progression associated with a disease or bodily condition. Disease progression can be monitored by clinical observations, laboratory and imaging investigations apparent to a person skilled in the art. A therapeutically effective amount can be an amount that is effective in a single dose or an amount that is effective as part of a multi-dose therapy, for example an amount that is administered in two or more doses or an amount that is administered chronically.

The effective amount of any one or more structures described herein may be from about 10 ng/kg of body weight to about 1000 mg/kg of body weight, and the frequency of administration may range from once a day to once a month. However, other dosage amounts and frequencies also may be used as the invention is not limited in this respect. A subject may be administered one or more structure described herein in an amount effective to treat one or more diseases or bodily conditions described herein.

An effective amount may depend on the particular condition to be treated. The effective amounts will depend, of course, on factors such as the severity of the condition being treated; individual patient parameters including age, physical condition, size and weight; concurrent treatments; the frequency of treatment; or the mode of administration. These factors are well known to those of ordinary skill in the art and can be addressed with no more than routine experimentation. In some cases, a maximum dose be used, that is, the highest safe dose according to sound medical judgment.

Actual dosage levels of the active ingredients in the pharmaceutical compositions described herein may be varied so as to obtain an amount of the active ingredient that is effective to achieve the desired therapeutic response for a particular patient, composition, and mode of administration, without being toxic to the patient.

The selected dosage level will depend upon a variety of factors including the activity of the particular inventive structure employed, the route of administration, the time of administration, the rate of excretion or metabolism of the particular structure being employed, the duration of the treatment, other drugs, compounds and/or materials used in combination with the particular structure employed, the age, sex, weight, condition, general health and prior medical history of the patient being treated, and like factors well known in the medical arts.

In some embodiments, a subject may be diagnosed with, or otherwise known to have, a disease or bodily condition associated with a NO mediated disorder. A NO mediated disorder is any disorder that is affected with NO therapy. NO mediated disorders include but are not limited to vascular conditions, diseases or disorders, as described herein. Vascular conditions, diseases or disorders include, but are not limited to, neurological disease, autoimmune disease, diseases of inflammation, diseases of blood vessels, angiogenesis, atherosclerosis, high blood pressure, kidney disease, cancer, cardiovascular disease, peripheral vascular disease, disease of the central nervous system, degenerative diseases, rheumatic diseases, connective tissue diseases, ischemia, tissue reperfusion, transplantation, infectious disease, thrombosis, diseases of blood clotting, hypercoagulation, platelet disorders, neutrophil disorders, disorders of white blood cells, endothelial disease, heart disease, erectile dysfunction, disorders of low blood flow and/or pulmonary disease. In some embodiments, the subject may be diagnosed with diseases related to cholesterol overload, revascularization, and/or in any case of where ischemia-reperfusion injury is suspected. In some embodiments, the subject may be diagnosed with , or otherwise known to have, a disease or bodily condition related to vascular injury, atherosclerosis, restenosis following vascular interventions (e.g. angioplasty), ischemia/reperfusion injury, ischemia-reperfusion injury following myocardial infarction and/or organ transplantation, prolong cold ischemia time of donor organs, atherosclerotic plaque burden, endothelial dysfunction and stiffening in atherosclerosis development, and/or disorders of blood pressure. In some embodiments, the subject may be diagnosed with, or otherwise known to have, a disease or bodily condition treated by precutaneous balloon angioplasty, stent placement, or disorders of blood vessel remodeling after procedures such as neointimal hyperplasia.

As used herein, a "subject" or a "patient" refers to any mammal (e.g., a human), for example, a mammal that may be susceptible to a disease or bodily condition such as a disease or bodily condition that is a vascular condition, disease or disorder. Examples of subjects or patients include a human, a non-human primate, a cow, a horse, a pig, a sheep, a goat, a dog, a cat or a rodent such as a mouse, a rat, a hamster, or a guinea pig. Generally, the invention is directed toward use with humans. A subject may be a subject diagnosed with a certain disease or bodily condition or otherwise known to have a disease or bodily condition. In some embodiments, a subject may be diagnosed as, or known to be, at risk of developing a disease or bodily condition. In some embodiments, a subject may be diagnosed with, or otherwise known to have, a vascular condition, disease or disorder, as described herein. In certain embodiments, a subject may be selected for treatment on the basis of a known disease or bodily condition in the subject. In some embodiments, a subject may be selected for treatment on the basis of a suspected disease or bodily condition in the subject. In some embodiments, the composition may be administered to prevent the development of a disease or bodily condition. However, in some embodiments, the presence of an existing disease or bodily condition may be suspected, but not yet identified, and a composition of the invention may be administered to diagnose or prevent further development of the disease or bodily condition.

A "biological sample," as used herein, is any cell, body tissue, or body fluid sample obtained from a subject. Examples of body fluids include, for example, lymph, saliva, blood, and urine. Samples of tissue and/or cells for use in the various methods described herein can be obtained through standard methods including, but not limited to, tissue biopsy, including punch biopsy and cell scraping, needle biopsy; or collection of blood or other bodily fluids by aspiration or other suitable methods.

The function and advantage of these and other embodiments will be more fully understood from the examples below. The following examples are intended to illustrate the benefits of the present invention, but do not exemplify the full scope of the invention. Accordingly, it will be understood that the example section is not meant to limit the scope of the invention.

### EXAMPLES

### Example 1

### Methods

DPPTE is dissolved in 100% ethanol, then diluted with water to 40% ethanol (60% water). HCl is added to adjust the pH to ~3. Sodium nitrite is dissolved in water, diluted to match the concentration of the DPPTE, then added to the DPPTE solution (20% ethanol final concentration). The metal chelator DTPA is added at a final concentration of 50 uM. The solution is vortexed and incubated at room temperature in the dark for ~1 hour. The reaction is stopped by neutralizing the acid (pH = 7) and the modified phospholipid is stored at -20 °C. The SNO DPPTE is purified by HPLC using a methanol:water gradient, lyophilized and dissolved in 100% ethanol. NO HDL NPs are synthesized using the same protocol as HDL NPs. 5nm citrate stabilized gold nanoparticles are surface functionalized by addition of 5 fold molar excess of apolipoprotein A1 for 1 hour at room temperature, followed by addition of 250-fold molar excess of the phospholipid PDP PE (disulfide containing phospholipid) and 250-fold molar excess SNO DPPTE. The nanoparticles are rocked over night at room temperature, then purified using tangential flow filtration (TFF).

### Synthesis of NO Liposome

SNO DPPTE was dried in a glass vial under nitrogen gas to create a thin film. Water was added to the vial, which was then sonicated for 10-30 minutes to produce liposomes of ~30-100nm in diameter. Liposomes were then stored at 4C in the dark.

### Drug coating of bare metal stents

The SNO DPPTE, NO Liposomes, HDL NPs and/or NO HDL NPs were combined with a biodegradable polymer and applied to a bare metal stent in a thin film.

### Intra-arterial injection of nitric oxide releasing compounds

The SNO DPPTE, NO Liposomes, HDL NPs and/or NO HDL NPs will be delivered following angioplasty using infusion catheters to localize the drugs to the site of vascular injury.

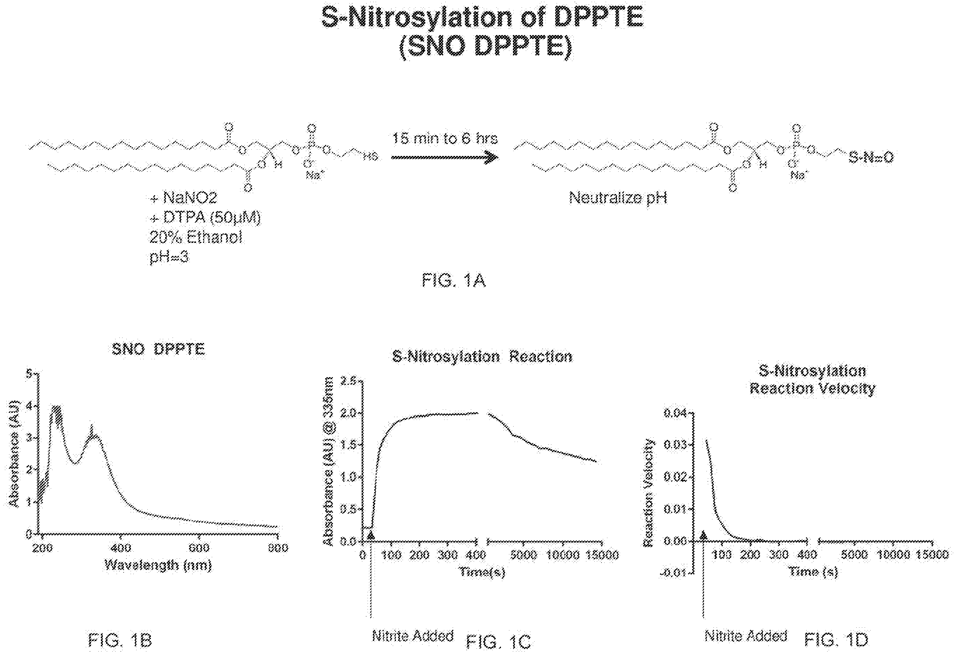

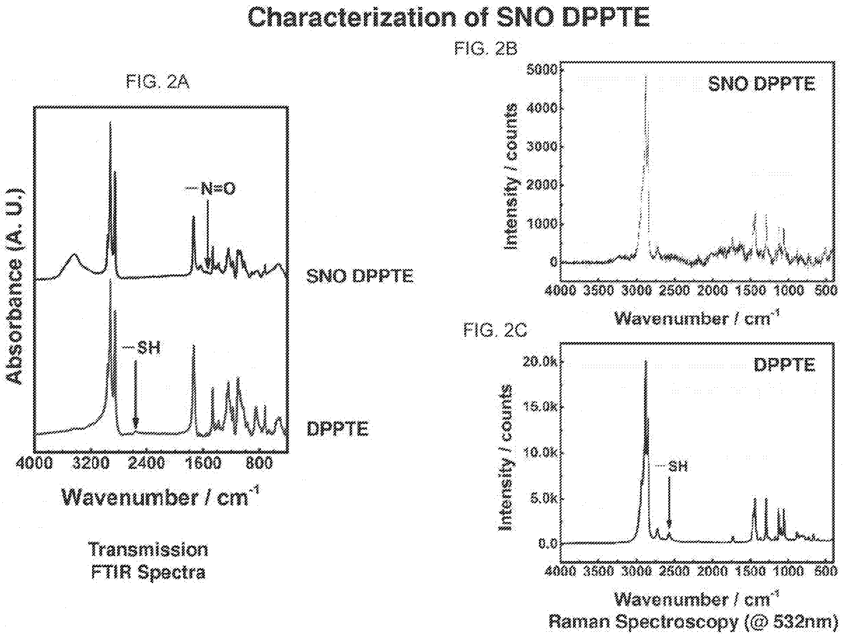

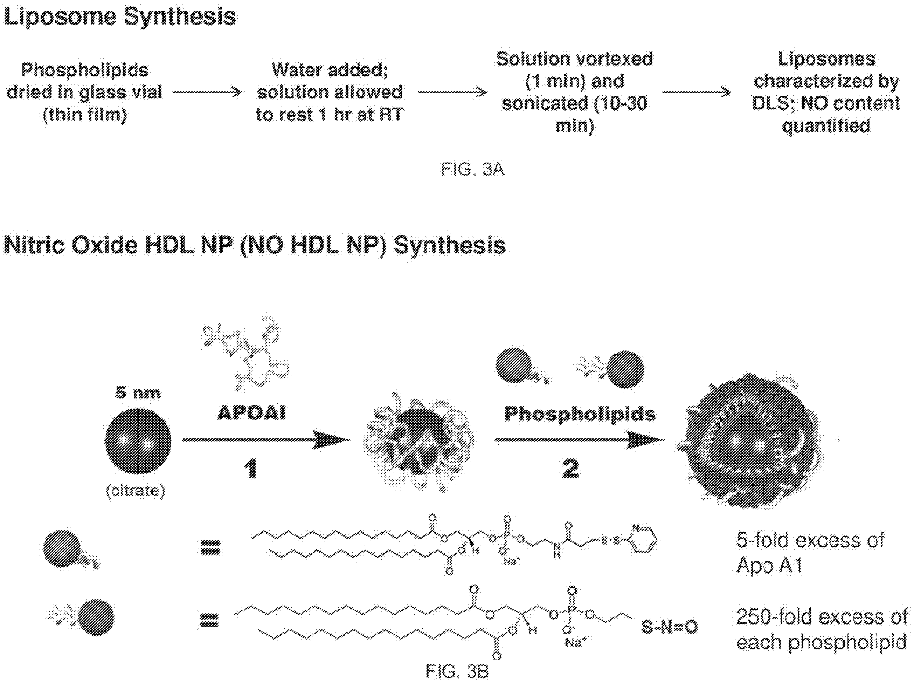

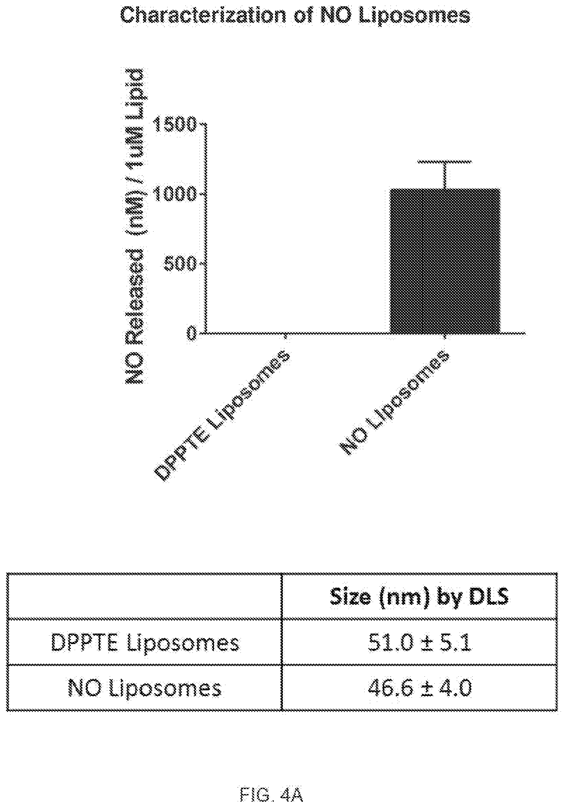

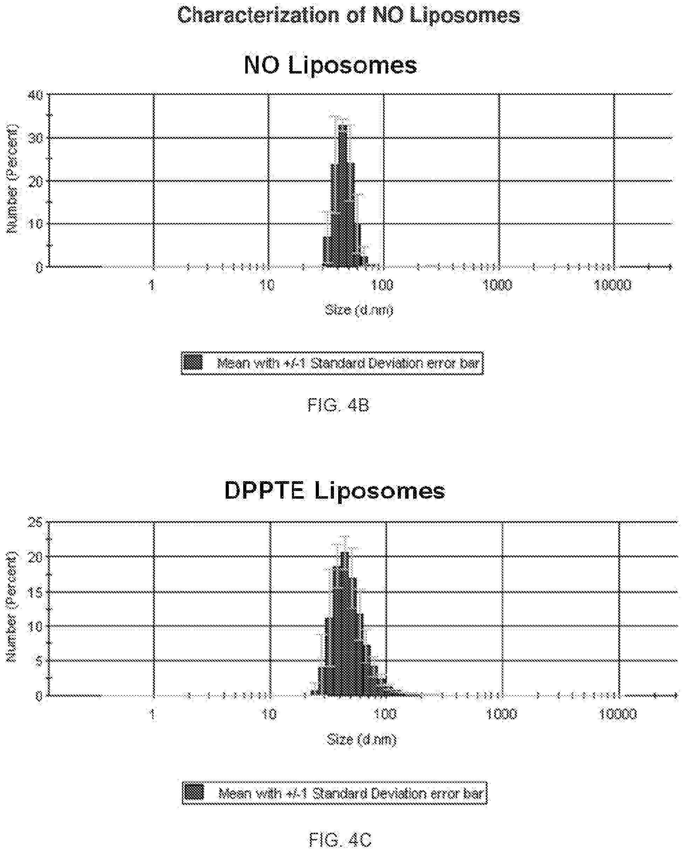

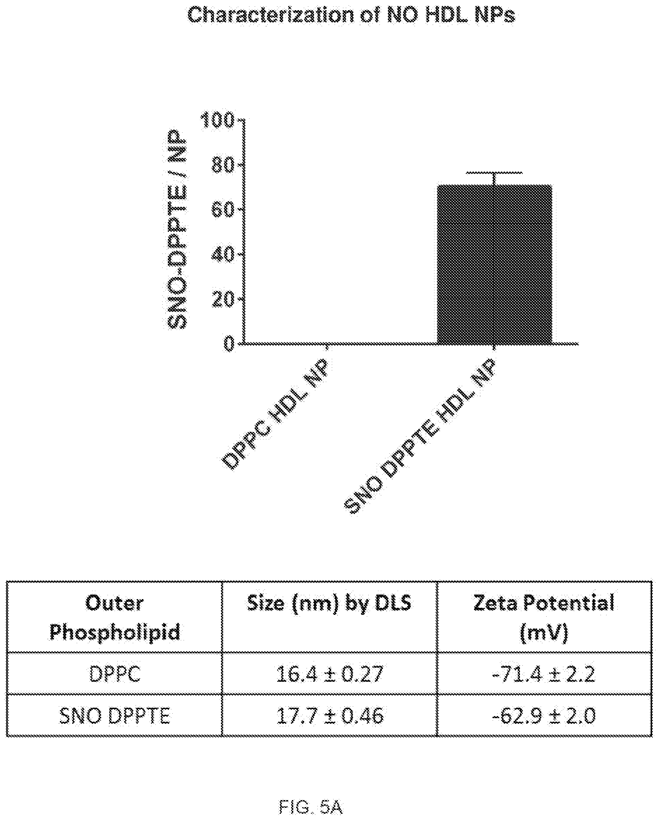

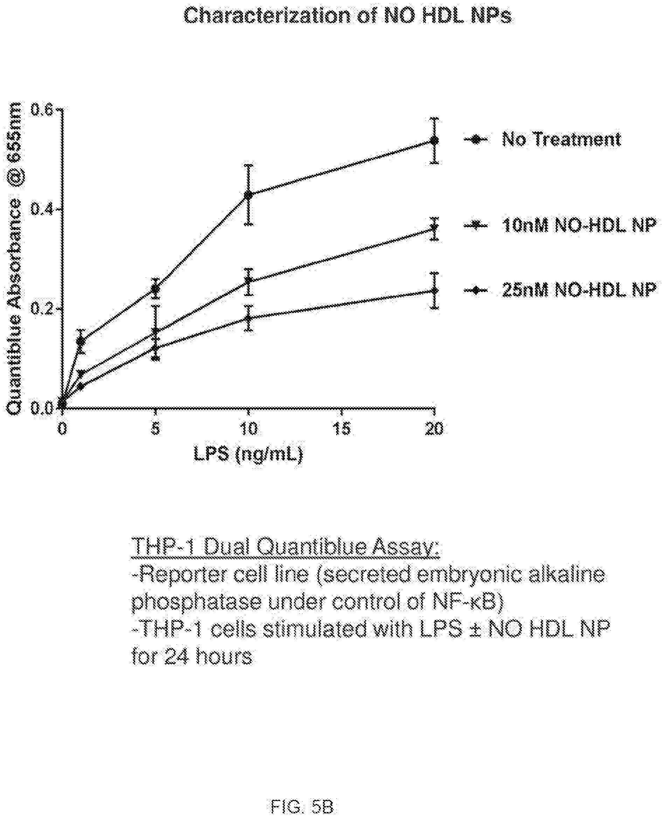

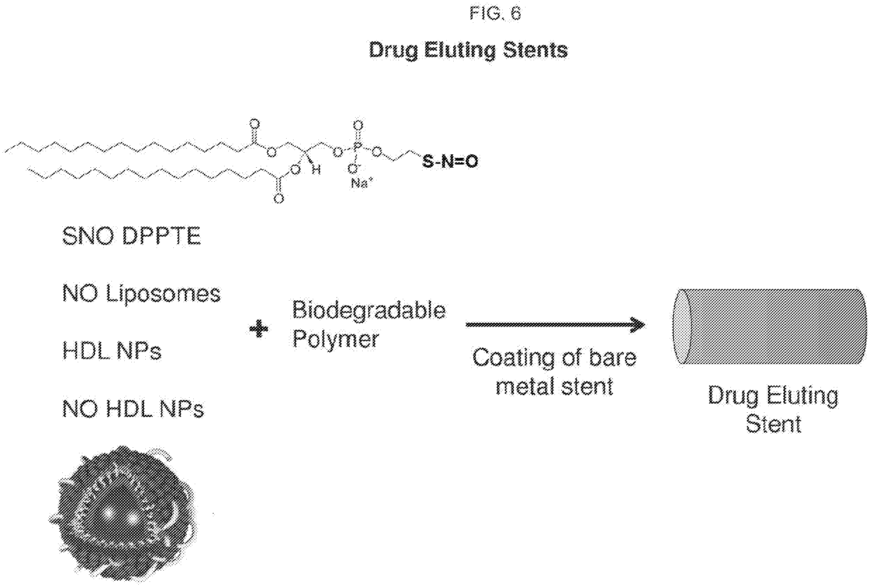

FIGs. 1-7 illustrate the modification of phospholipid reaction, NO Liposome synthesis, HDL NP synthesis, NO HDL NP synthesis, experimental data demonstrating the presence of the SNO group on SNO DPPTE, the presence of SNO DPPTE phospholipid on NO HDL NPs, the presence of SNO in NO Liposomes, NO release in a biological system (in vitro), and the formulation of phospholipid-, liposome- and nanoparticle-loaded stents.

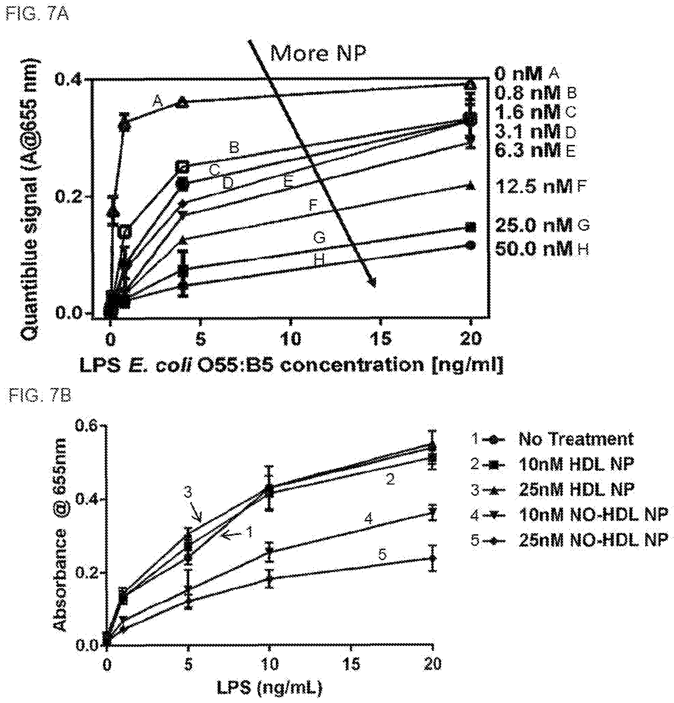

The ability of SNO-HDL NPs to deliver nitric oxide in an *in vitro* or *in vivo* setting was tested on ischemia-reperfusion injury. During ischemia-reperfusion injury, NO levels decrease in the affected tissue resulting in activation of toll-like receptor 4 (TLR4), inflammatory cell infiltration, and extensive cellular damage. To test the SNO-HDL NPs *in vitro,* we utilized the THP-1 dual reporter cell line, a human monocyte cell line expressing a secreted embryonic alkaline phosphatase reporter gene downstream of a promoter containing the NF-κB consensus binding sequence. Stimulation of the cells via TLR4 leads to NF-κB activation, and expression of the reporter gene, which can then be visualized in a simple colorimetric assay. Lipopolysaccharide (LPS) stimulation of THP-1 dual cells resulted in significant expression of the reporter gene (FIGs. 7A and 7B). HDL NPs, composed of standard disulfide containing inner phospholipid PDP PE and cardiolipin (18:2 PG) as the outer phospholipid, dose-dependently decreased LPS-induced inflammation (FIG. 7A). Similarly, SNO-HDL NPs demonstrated a dose-dependent inhibitory effect on reporter gene expression (FIGs. 7A and 7B), strongly suggesting that the SNO-HDL NPs released NO in sufficient quantities to prevent an LPS-induced inflammatory response.

### Example 2: Synthesis of High-Density Lipoprotein-Like Nanoparticles for Nitric Oxide Delivery With Application to Ischemia/ Reperfusion Injury

### Methods

### Preparation of S-nitrosylated 1,2-dipalmitoyl-sn-glycero-3-phosphothioethanol (SNO-PL).

A commercially available thiol containing phospholipid DPPTE was S-nitrosylated through addition of sodium nitrite under acidic conditions. DPPTE was reconstituted in 100% ethanol to a concentration of 25mM then diluted with water to a final concentration of 5mM DPPTE, in 20% ethanol/80%water. The pH of the solution was lowered to 3 by addition of HCl. Pentatonic acid (DPTA) was added at a final concentration of 50uM, to chelate any heavy metal ions that may be present. The S-N=O group is particularly susceptible to degradation by heavy metals such as copper and zinc, necessitating the strong chelating agent DPTA. Finally, sodium nitrite was added to the solution and the reaction tracked using UV/Vis spectroscopy. The S-N=O group has an absorbance maximum at 335nm, and the accumulation of the S-nitrosylated product can be monitored over time. The typical ratio of DPPTE to sodium nitrite was 1:1 unless otherwise stated. UV/Vis spectroscopy was used both to characterize the end product as well as monitor reaction progression over time.

### Characterization of SNO-PL.

Following synthesis, the SNO-PL was characterized by mass spectroscopy, FTIR, Raman spectroscopy, and UV/Vis spectroscopy. For mass spectroscopy, a small sample of each reaction (e.g., different ratio of phospholipid to sodium nitrite) was run on the mass spectrometer in the Peptide Synthesis Core at Northwestern University. FTIR and Raman spectroscopy was performed at Northwestern University's Keck Facility. SNO-PL was first dried under nitrogen prior to analysis.

### High Density Lipoprotein-Like Nanoparticle Synthesis.

HDL NP synthesis was carried out as previously described. Briefly, 5nm citrate stabilized gold nanoparticles were surface functionalized with a 5-fold molar excess of apolipoprotein A1 and a phospholipid bilayer, with each phospholipid added at a 250-fold molar excess relative to the gold nanoparticle concentration. The disulfide containing phospholipid PDP PE was used as the inner phospholipid in all syntheses. The outer phospholipid of the HDL NPs was a combination of DPPC and the SNO-PL. Following an overnight incubation, the HDL NPs were then subjected to tangential flow filtration. The concentration of the HDL NPs was determined using Beer's law and UV/Vis spectroscopy. The various constructs were analyzed for their size (dynamic light scattering; DLS) and surface charge (zeta potential), using the Malvern zetasizer. Nitric oxide content and long-term stability of the SNO group was assayed using the Sievers Nitric Oxide analyzer (NOA) and the tri-iodine method, described previously. Briefly, a solution of iodine and iodide was mixed with glacial acetic acid and loaded into the NOA. SNO HDL NP samples were injected into the solution, which then released any still bound nitric oxide into the instrument, where it combined with oxygen to produce a chemiluminescent signal. Samples of the SNO HDL NPs were taken over time, and the percentage of SNO groups remaining reported here.

### Toxicity.

The MTS assay was used to quantify the toxicity of SNO HDL NPs and HDL NPs on human aortic endothelial cells and human arterial smooth muscle cells. Cells were plated at 1^{∗}10⁵ cells/ml into 96 well plates, and were treated with HDL NPs or SNO HDL NPs for 48 hours prior to addition of the MTS reagent. A Biotek Synergy 2 plate reader was used to measure the absorbance at 490nm prior to MTS reagent addition, at time = 0, and at time = 120 minutes. Percent viability was calculated by subtracting the time = 0 values from the time = 120 values, then standardizing the resultant values to the PBS control (set to 100%).

### Transwell Migration Assay.

AoSMCs were resuspended at a concentration of 1^{∗}10⁶ cells/ml, and 100µl of cells was added to the interior of an 8µm pore size transwell insert placed in a 24 well plate. The cells were incubated for 10 minutes to allow for attachment, then 600µl of culture media + treatment was added. HDL NPs and SNO HDL NPs were added at a final concentration of 50nM. The cells were incubated for 4 hours, then washed twice with PBS, and fixed with 100% ethanol. Following fixation, the cells were stained with crystal violet and the number of cells migrating through the insert calculated by averaging 10 fields per replicate.

### Murine Kidney Transplantation Model.

The murine kidney transplant model was performed as described previously. Donor C57/B16 mice were injected with 100µl of PBS, 1µM HDL NPs or 1µM SNO HDL NPs 2 hours prior to harvesting of the donor kidney. The kidney was resected, along with a portion of the aorta and inferior vena cava, perfused with a cold solution of 250nM HDL NP or SNO HDL NP in University of Wisconsin (UW) solution. The donor organ was transferred to 4oC for 4 hours prior to transplantation into the recipient C57/B16 mouse. The recipient mouse underwent a bilateral nephrectomy, with the first native kidney removed prior to transplantation and the second native kidney removed following transplantation. The transplanted kidney is connected to the vasculature using the aorta and vena cava segments retained from the donor. Following transplantation, mice were treated with either 100µl of PBS, 1µM HDL NP or 1µM SNO HDL NP intraperitoneally. The following day, the recipients received an additional dose, this time via tail vein. Blood was collected on Day 2 and analyzed for plasma creatinine level. The transplanted organs were also resected, fixed with formalin, embedded in O.C.T. and processed by the Mouse Histology and Phenotyping Laboratory at Northwestern University to investigate infiltration of the grafts by immune cells (e.g Gr-1), apoptosis (TUNEL staining), proliferation (Ki67) and gross histology (H&E).

### Fluorescent Microscopy.

The Nikon A1R GaAsP confocal fluorescent microscope in the Center for Advanced Microscopy/ Nikon Imaging Facility at Northwestern University was used to image immunocytochemical stained kidney sections.

### Introduction

Nitric oxide (NO) is a powerful vasodilator and second messenger involved in cell signaling. However, due to its high reactivity, NO has an extremely short half-life, rendering delivery problematic. In biological systems, S-nitrosylation of free thiols increases the half-life of NO. An S-nitrosylated phospholipid was synthesized, characterized and incorporated into bio-inspired high-density lipoprotein-like nanoparticles (SNO HDL NPs). S-nitrosylation was achieved by adding sodium nitrite to a thiol-containing phospholipid under acidic conditions. This reaction led to rapid S-nitrosylation of the thiol-containing phospholipid. The SNO-PL was used to synthesize SNO HDL NPs, whereby the amount of NO on the HDL NP could be easily tailored. SNO HDL NPs retained NO when stored at 4°C for at least 100 days. *In vivo,* SNO HDL NPs reduced ischemia/reperfusion injury in a mouse kidney transplant model. These results detail the synthesis of SNO-PL, SNO HDL NPs, and proof-of-principle of the ability of SNO HDL NPs to deliver therapeutic quantities of NO and reduce ischemia/reperfusion injury.

NO is a gaseous molecule with potent biological effects. NO is a potent vasodilator and mediates intracellular signaling¹. Altered NO levels have been implicated in a variety of disorders, including sickle cell disease, erectile dysfunction, rheumatoid arthritis, atherosclerosis, and ischemia/reperfusion injury (IRI).

Since NO exists as a free radical gas, its half-life in biological systems is extremely short, on the order of milliseconds or less. Most NO delivery methods utilize an NO donor, such as a diazeniumdiolate, or involve the use of inhaled nitric oxide gas². However, these compounds suffer from short half-lives, and unfavorable biodistribution patterns. Several nanoparticles have been developed as delivery platforms for NO³. While some metal/metal oxide nanoparticles have been developed to deliver nitric oxide⁴⁻⁶, the majority of research has focused on silica nanoparticles. Generally speaking, these silica nanoparticles are functionalized with diazeniumdiolates as a method to release NO, and have been shown to decrease blood pressure, increase vasodialation and ameliorate hemoglobin-induced vasoconstriction in hamsters^{7,8}. With respect to ischemialreperfusion injury, conjugation of the NO donor SNAP (S-nitroso-N-acetyl-D,L-penicillamine) to a dendrimer nanoparticulate scaffold reduced the size of infarction injury in explanted rat hearts⁹. While these results are promising, significant limitations of silica and dendrimeric nanoparticles, including stability in aqueous solutions, release of nitric oxide prior to injection, relatively poor stability of diazeniumdiolates, and a lack of targeting, render these nanostructures poorly suited for *in vivo* applications.

The synthesis and characterization of high-density lipoprotein-like nanoparticles (HDL NPs), which mimic the size, shape, surface composition, and some functions of natural HDLs, has been previously described.¹⁰⁻¹² HDL NPs may be composed of a 5 nm gold nanoparticle core, surface functionalized with the HDL-defining apolipoprotein A-I, and a phospholipid bilayer. Incorporation of an S-nitrosylated phospholipid (SNO-PL) into the bilayer of HDL NPs was hypothesized to allow for the delivery of NO both *in vitro* and *in vivo.*

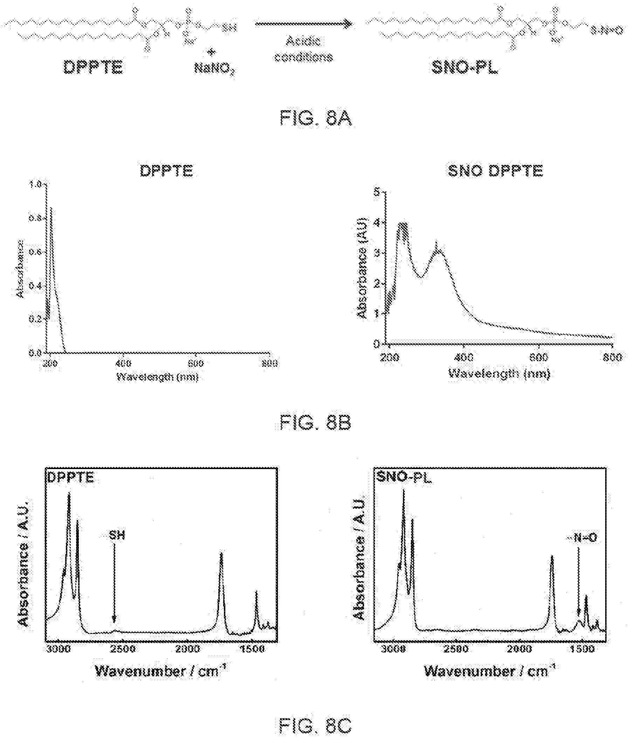

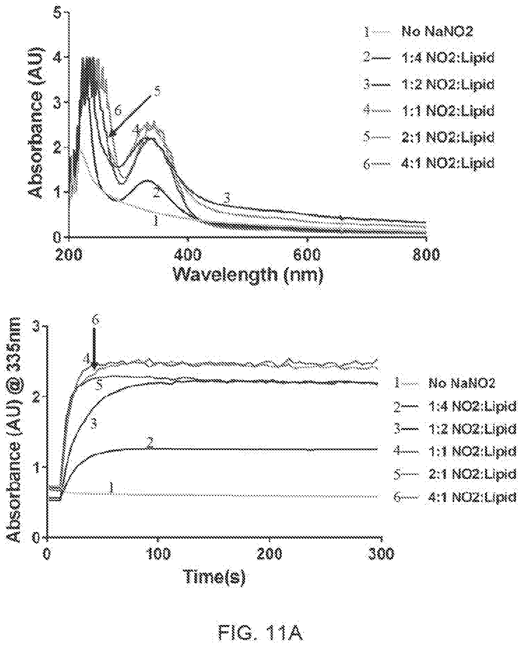

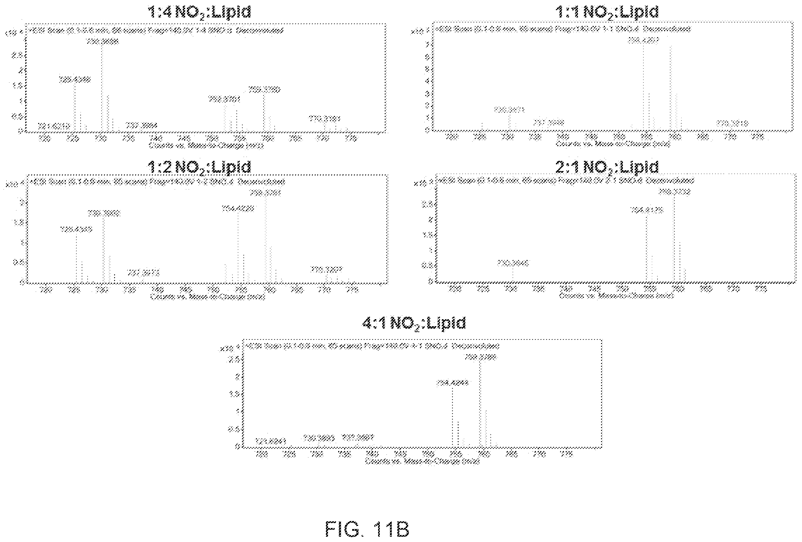

The commercially available, thiol-containing phospholipid 1,2-dipalmitoyl-*sn-*glycero-3-phosphothioethanol (DPPTE) was employed. This PL was S-nitrosylated by addition of an equimolar quantity of NaNO₂ under acidic (pH=3) conditions in a 20% ethanol/80% water (v/v) solution (FIG. 8A). The -S-N=O moiety has an absorbance maximum at 335 nm, which allows for reaction monitoring by UV/Vis spectroscopy. Addition of NaNO₂ (FIG. 8B) to an equimolar concentration of the phospholipid resulted in complete and rapid conversion of DPPTE to SNO-PL (FIG. 8C, FIG. 11A). Altering the ratio of NaNO₂ to DPPTE did not result in increased S-nitrosylation or faster reaction kinetics (FIG. 11A). Mass spec analysis demonstrated that equimolar addition of NaNO₂ to DPPTE resulted in almost complete S-nitrosylation of the free thiol group (FIG. 11B). Increasing the quantity of NaNO₂ did not result in further S-nitrosylation (FIG. 11B).

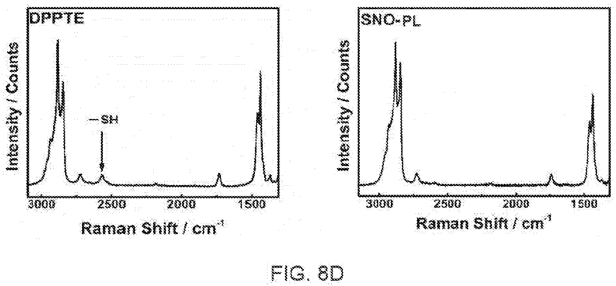

FTIR and Raman spectroscopy of SNO-PL and DPPTE further confirmed the transformation of the -S-H group to an -S-N=O moiety (FIG. 8C, FIG. 8D). FTIR spectra of SNO-PL show a peak for the -N=O moiety at approximately 1530 wavenumber, which is not present in the DPPTE spectra (FIG. 8C). Conversely, in the Raman spectra, a peak for the S-H bond at 2580 wavenumber is present in the DPPTE spectra but absent in the SNO-PL spectra (FIG. 8D).

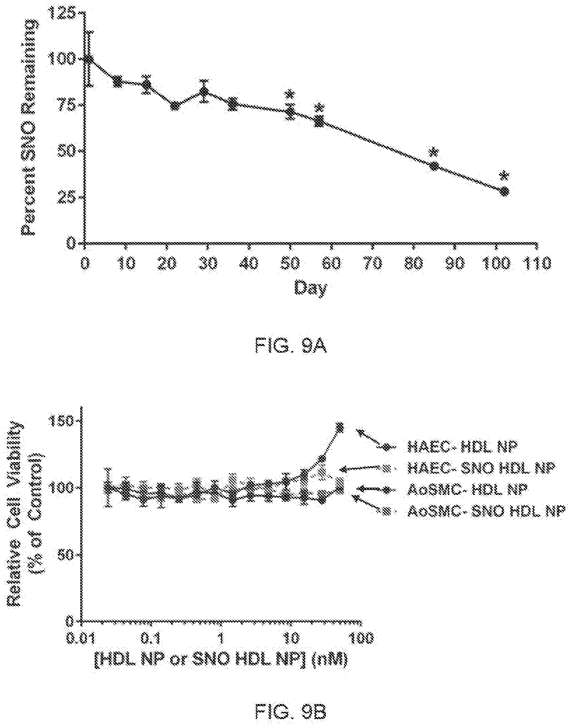

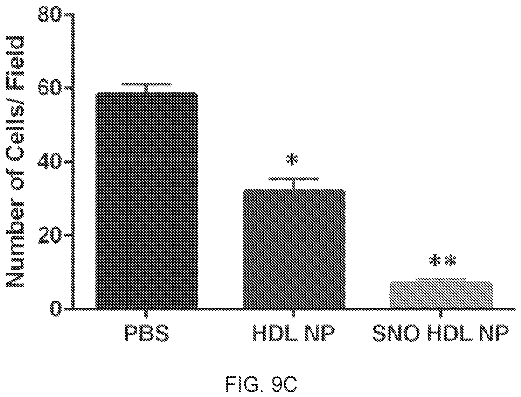

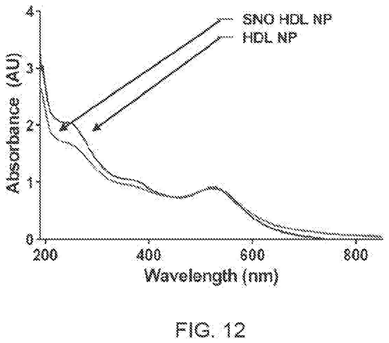

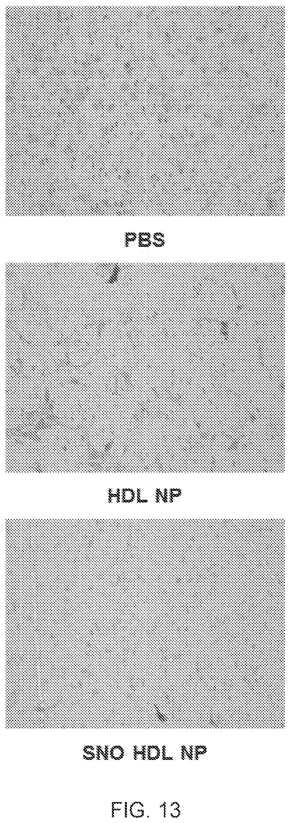

SNO HDL NP synthesis was carried out using standard protocols, with gold colloid, apoA-I and phospholipids in a 20% ethanol/80% water (v/v) solution. The conjugates were purified by tangential flow filtration, as previously described¹⁰⁻¹². UV/Vis spectroscopy of SNO HDL NPs was similar to spectra for HDL NPs, with a local maximum at ~520nm corresponding to the surface plasmon resonance of the gold nanoparticle. Due to signal from the gold nanoparticle and apoA-I, no peak at 335nm was detected in the SNO HDL NPs (FIG. 12). Chemiluminescent detection, using a Sievers Nitric Oxide Analyzer (NOA), and a solution of I3- in glacial acetic acid¹³, demonstrated the presence of the SNO groups on SNO HDL NPs (FIG. 9A). The SNO groups were stable on the SNO HDL NPs, when stored at 4°C, with 71.4% ± 3.9% SNO remaining after 50 days, and 28.4% ± 1.3% after 100 days (FIG. 9A). SNO HDL NP and HDL NP toxicity towards human aortic endothelial cells (HAEC) and human aortic smooth muscle cells (AoSMC), two of the expected cell types to interact with the nanoparticles, was quantified using the MTS assay. Both nanoparticle constructs were non-toxic in the HAECs and AoSMCs (FIG. 9B). To verify that the SNO HDL NPs could successfully deliver a physiologically relevant dose of nitric oxide, we tested the ability of the SNO HDL NPs to inhibit migration of aortic smooth muscle cells. Nitric oxide has been shown to inhibit the migration of smooth muscle cells, both *in vitro* and *in vivo.* SNO HDL NPs significantly inhibited AoSMC migration in a transwell migration assay (FIG. 9C; FIG. 13). Interestingly, the HDL NP construct partially inhibited AoSMC migration, suggesting that the inherent functionality of the HDL NP beyond the ability to deliver NO.

Ischemia/reperfusion injury (IRI), defined as a period of hypoxia or anoxia followed by reintroduction of oxygen, plays a critical role in a number of different pathologies, from myocardial infarction and stroke, to damage of transplanted organs and tissues¹⁴⁻¹⁸. In the case of transplantation, donor organs experience two distinct phases of ischemia: an acute period of warm ischemia, from the time of complete occlusion of blood flow (i.e. cross-clamp) to organ harvest, and then a more prolonged period of cold ischemia, where the organ is perfused with cold preservation solution, transported, and eventually transplanted into the recipient^{19,20}. Following transplantation, the donor organ undergoes reperfusion, where the sudden influx of oxygen exacerbates ischemic damage, which can lead to delayed graft function, among other things. With the scarcity of donor organs, maximizing graft function is critical, especially those from marginal donors. Nitric oxide plays a significant role in protecting cells from IRI; however, prolonged periods of ischemia leads to decreased expression and activity of endothelial nitric oxide synthase (eNOS) in endothelial cells . Restoration of NO levels, through delivery of exogenous NO, may ameliorate IRI and improve graft function.

To investigate the ability of the SNO HDL NPs and HDL NPs to ameliorate IRI in kidney transplantation *in vivo,* we utilized a mouse kidney transplant model. IRI is one of the major contributors to delayed graft function, a relatively common complication that presents immediately post transplantation and factors in determining the long-term outcome of the transplanted organ²²⁻²⁴.

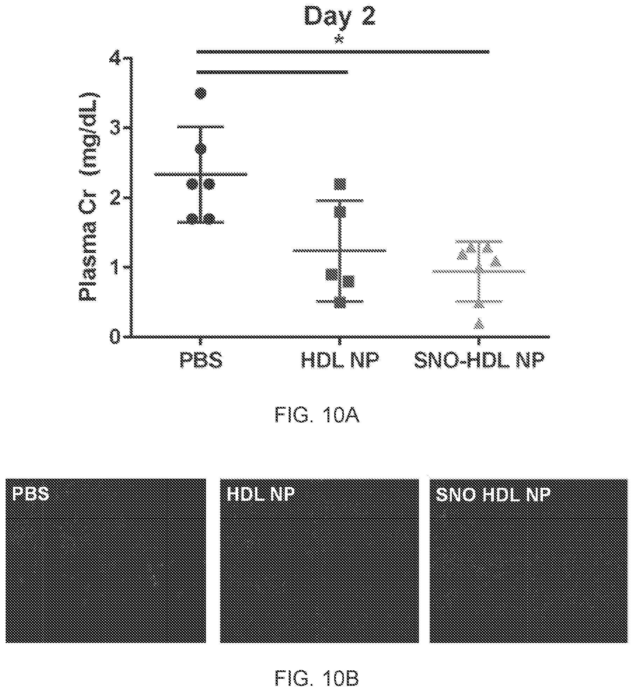

During the process of IRI in allogeneic kidney transplants, the innate and adaptive immune systems are activated, leading to infiltration of the kidney graft by host immune cells^{19, 25-27}. The acute inflammatory response increases the immunogenicity of the transplanted graft, potentially leading to graft rejection. Numerous strategies exist to mitigate IRI, including, strict selection of the donor, minimized cold ischemia time, and administration of anti-inflammatory drugs²⁰. Kidneys were harvested from donor mice, placed on ice for 4 hours, and then transplanted to a recipient mouse that has undergone a bilateral nephrectomy, leaving the transplanted kidney graft as the only functional kidney remaining. Donors were treated with nanoparticles prior to organ harvest, the organ perfused with nanoparticles during cold ischemia incubation, and the recipient mouse treated with nanoparticles immediately following surgery and again 24 hours later. Plasma creatinine was measured on day 2. HDL NP and SNO HDL NP both decreased plasma creatinine levels compared to controls (2.333 ± 0.683 mg/dL for PBS treated v. 1.240 ± 0.723 for HDL NP treated v. 0.943 ± 0.428 for SNO HDL NP treated; p<0.05 v. PBS treated v. nanoparticles; FIG. 10A).

Interestingly, the HDL NP construct itself ameliorated some of the IRI damage, suggesting that HDL NPs have an inherent ability to protect kidney tissue from IRI. It should be noted that in this transplant model, plasma creatinine levels returned to baseline values around day 14, a time line that is far shorter than in human kidney transplant recipients.

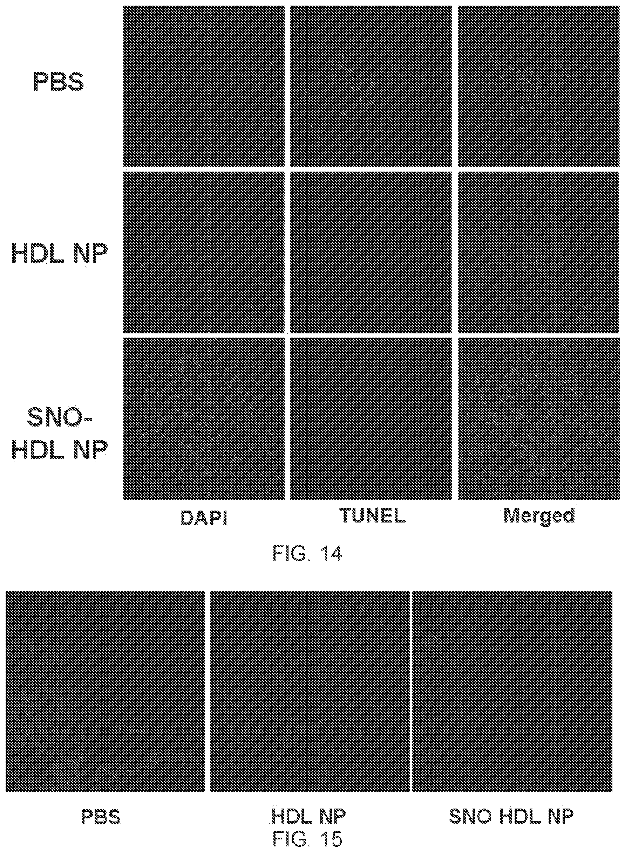

Immunocytochemical staining for apoptosis (TUNEL) and proliferation (Ki67) demonstrated that HDL NP and SNO HDL NP both decreased the number of apoptotic cells and increased the number of proliferating cells (FIG. 14). Macrophage infiltration in the grafts was similar across all treatment groups (FIG. 15). Infiltration by neutrophils, visualized by staining the kidney grafts for Gr-1, was reduced in SNO HDL NPs compared to HDL NP and PBS controls (FIG. 10B). These data suggest that the HDL NP construct itself acts to prevent apoptosis and induce proliferation in renal cells, while the nitric oxide delivered by the SNO HDL NP limited infiltration of the transplanted kidney by neutrophils.

### Conclusion

High density lipoprotein-like nanoparticles can be successfully loaded with an S-nitrosylated phospholipid, resulting in a nitric oxide-releasing HDL NP that is stable, non-toxic, and capable of delivering a physiologically relevant dose of NO both *in vitro* and in an *in vivo* model of kidney transplantation. Such formulations can be coated onto or otherwise integrated into an implantable device.

### References

(1) Schulz, R.; Kelm, M.; Heusch, G. Cardiovascular research 2004, 61, 402.
(2) Deshpande, S. R.; Satyanarayana, K.; Rao, M. N.; Pai, K. V. Indian journal of pharmaceutical sciences 2012, 74, 487.
(3) Quinn, J. F.; Whittaker, M. R.; Davis, T. P. Journal of controlled release : official journal of the Controlled Release Society 2015, 205, 190.
(4) Polizzi, M. A.; Stasko, N. A.; Schoenfisch, M. H. Langmuir : the ACS journal of surfaces and colloids 2007, 23, 4938.
(5) Rothrock, A. R.; Donkers, R. L.; Schoenfisch, M. H. J Am Chem Soc 2005, 127, 9362.
(6) Zhang, X. F.; Mansouri, S.; Mbeh, D. A.; Yahia, L.; Sacher, E.; Veres, T. Langmuir : the ACS journal of surfaces and colloids 2012, 28, 12879.
(7) Cabrales, P.; Han, G.; Nacharaju, P.; Friedman, A. J.; Friedman, J. M. American journal of physiology. Heart and circulatory physiology 2011, 300, H49.
(8) Cabrales, P.; Han, G.; Roche, C.; Nacharaju, P.; Friedman, A. J.; Friedman, J. M. Free radical biology & medicine 2010, 49, 530.
(9) Johnson, T. A.; Stasko, N. A.; Matthews, J. L.; Cascio, W. E.; Holmuhamedov, E. L.; Johnson, C. B.; Schoenfisch, M. H. Nitric oxide : biology and chemistry / official journal of the Nitric Oxide Society 2010, 22, 30.
(10) Luthi, A. J.; Lyssenko, N. N.; Quach, D.; McMahon, K. M.; Millar, J. S.; Vickers, K. C.; Rader, D. J.; Phillips, M. C.; Mirkin, C. A.; Thaxton, C. S. Journal of lipid research 2015.
(11) Luthi, A. J.; Zhang, H.; Kim, D.; Giljohann, D. A.; Mirkin, C. A.; Thaxton, C. S. ACS Nano 2012, 6, 276.
(12) Thaxton, C. S.; Daniel, W. L.; Giljohann, D. A.; Thomas, A. D.; Mirkin, C. A. J Am Chem Soc 2009, 131, 1384.
(13) MacArthur, P. H.; Shiva, S.; Gladwin, M. T. Journal of chromatography. B, Analytical technologies in the biomedical and life sciences 2007, 851, 93.
(14) Bagheri, F.; Khori, V.; Alizadeh, A. M.; Khalighfard, S.; Khodayari, S.; Khodayari, H. Life sciences 2016, 165, 43.
(15) Bonventre, J. V.; Yang, L. The Journal of clinical investigation 2011, 121, 4210.
(16) Munshi, R.; Hsu, C.; Himmelfarb, J. BMC medicine 2011, 9, 11.
(17) Piper, H. M.; Meuter, K.; Schafer, C. The Annals of thoracic surgery 2003, 75, S644.
(18) Yellon, D. M.; Hausenloy, D. J. The New England journal of medicine 2007, 357, 1121.
(19) Eltzschig, H. K.; Eckle, T. Nature medicine 2011, 17, 1391.
(20) Salvadori, M.; Rosso, G.; Bertoni, E. World journal of transplantation 2015, 5, 52.
(21) Giraldez, R. R.; Panda, A.; Xia, Y.; Sanders, S. P.; Zweier, J. L. The Journal of biological chemistry 1997, 272, 21420.
(22) Cooper, J. E.; Wiseman, A. C. Current opinion in nephrology and hypertension 2013, 22, 698.
(23) Ortiz, F.; Paavonen, T.; Tornroth, T.; Koskinen, P.; Finne, P.; Salmela, K.; Kyllonen, L.; Gronhagen-Riska, C.; Honkanen, E. Journal of the American Society of Nephrology : JASN 2005, 16, 817.
(24) Ponticelli, C. Nephrology, dialysis, transplantation : official publication of the European Dialysis and Transplant Association - European Renal Association 2014, 29, 1134.
(25) Denecke, C.; Tullius, S. G. Progres en urologie : journal de l'Association francaise d'urologie et de la Societe francaise d'urologie 2014, 24 Suppl 1, S13.
(26) Erpicum, P.; Detry, O.; Weekers, L.; Bonvoisin, C.; Lechanteur, C.; Briquet, A.; Beguin, Y.; Krzesinski, J. M.; Jouret, F. Nephrology, dialysis, transplantation : official publication of the European Dialysis and Transplant Association - European Renal Association 2014, 29, 1487.
(27) Jang, H. R.; Rabb, H. Nature reviews. Nephrology 2015, 11, 88.

The present invention is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present invention.

Furthermore, the invention encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, e.g., in Markush group format, each subgroup of the elements is also disclosed, and any element(s) can be removed from the group. It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth in haec verba herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," are to be understood to be open-ended, i.e., to mean including but not limited to.

## Claims

1. An implantable device comprising a nanostructure of reservoir molecules that are reservoirs for nitric oxide;
wherein the reservoir molecule is a S-Nitrosylated lipid or a S-Nitrosylated phospholipid such as S-Nitrosylated 1,2-dipalmitoyl-sn-glycero-3-phosphothioethanol (DPPTE); and
wherein the nanostructure has a gold core and a lipid bilayer or monolayer.

2. The implantable device of claim 1, further comprising an apolipoprotein, optionally apolipoprotein A-I (apoA-I).

3. The implantable device of claim 1 or 2, wherein the implantable device is a stent, and optionally a drug-eluting stent.

4. A nanostructure of reservoir molecules that are reservoirs for nitric oxide for use in a method for delivering NO to a subject having a NO mediated disorder, the method comprising:
administering by injection, through a catheter or on an implantable device, of the nanostructure of reservoir molecules,
wherein the reservoir molecules are a S-Nitrosylated lipid or a S-Nitrosylated phospholipid such as S-Nitrosylated 1,2-dipalmitoyl-sn-glycero-3-phosphothioethanol (DPPTE);
and wherein the nanostructure has a gold core and a lipid bilayer or monolayer.

5. The nanostructure of reservoir molecules according to claim 4, wherein the NO mediated disorder is angiogenesis, ischemia-reperfusion, restenosis, angioplasty, atherosclerosis, dysregulated blood pressure or graft rejection after transplantation.

6. The nanostructure of reservoir molecules of claim 4 or 5, wherein the nanostructure further comprises an apolipoprotein, optionally apolipoprotein A-I (apoA-I).

7. A S-Nitrosylated lipid or a S-Nitrosylated phospholipid such as S-Nitrosylated 1,2-dipalmitoyl-sn-glycero-3-phosphothioethanol (DPPTE) for use in a method for transplanting a donor organ in a recipient subject, the method comprising:
harvesting a donor organ;
contacting the donor organ with the implantable device of any one of claims 1-3; and
transplanting the donor organ into a recipient subject, wherein the implantable device reduces the risk of rejection of the donor organ relative to the risk of a donor organ transplanted without exposure to the implantable device.

8. A S-Nitrosylated lipid or a S-Nitrosylated phospholipid such as S-Nitrosylated 1,2-dipalmitoyl-sn-glycero-3-phosphothioethanol (DPPTE) for use in a method for transplanting an organ in a recipient subject, the method comprising:
preparing an implantable device that is an organ; wherein the implantable device is a device of any one of claims 1-3; and transplanting the organ into a recipient subject.

## Patentansprüche

1. Implantierbare Vorrichtung, umfassend eine Nanostruktur von Reservoirmolekülen, die Reservoirs für Stickstoffmonoxid sind;
worin das Reservoirmolekül ein S-Nitrosyliertes Lipid oder ein S-Nitrosyliertes Phospholipid, wie S-Nitrosyliertes 1,2-Dipalmitoyl-sn-glycero-3-phosphothioethanol (DPPTE), ist; und
worin die Nanostruktur einen Goldkern und eine Lipid-Doppelschicht oder -Monoschicht aufweist.

2. Implantierbare Vorrichtung gemäß Anspruch 1, ferner umfassend ein Apolipoprotein, optional Apolipoprotein A-I (apoA-I).

3. Implantierbare Vorrichtung gemäß Anspruch 1 oder 2, worin die implantierbare Vorrichtung ein Stent, und optional ein medikamentenfreisetzender Stent ist.

4. Nanostruktur von Reservoirmolekülen, die Reservoirs für Stickstoffmonoxid sind, zur Verwendung in einem Verfahren zur Abgabe von NO an ein Subjekt mit einer NOvermittelten Störung, das Verfahren umfassend:
Verabreichung der Nanostruktur von Reservoirmolekülen durch Injektion über einen Katheter oder auf einer implantierbaren Vorrichtung,
worin die Reservoirmoleküle ein S-Nitrosyliertes Lipid oder ein S-Nitrosyliertes Phospholipid, wie S-Nitrosyliertes 1,2-Dipalmitoyl-sn-glycero-3-phosphothioethanol (DPPTE), sind;
und worin die Nanostruktur einen Goldkern und eine Lipid-Doppelschicht oder -Monoschicht aufweist.

5. Nanostruktur von Reservoirmolekülen gemäß Anspruch 4, worin die NO-vermittelte Störung Angiogenese, Ischämie-Reperfusion, Restenose, Angioplastie, Atherosklerose, dysregulierter Blutdruck oder Transplantatabstoßung nach Transplantation ist.

6. Nanostruktur von Reservoirmolekülen gemäß Anspruch 4 oder 5, worin die Nanostruktur ferner ein Apolipoprotein, optional Apolipoprotein A-I (apoA-I), umfasst.

7. S-Nitrosyliertes Lipid oder S-Nitrosyliertes Phospholipid, wie S-Nitrosyliertes 1,2-Dipalmitoyl-sn-glycero-3-phosphothioethanol (DPPTE), zur Verwendung in einem Verfahren zur Transplantation eines Spenderorgans in einen Empfänger, das Verfahren umfassend:
Entnahme eines Spenderorgans;
Inkontaktbringen des Spenderorgans mit der implantierbaren Vorrichtung gemäß einem der Ansprüche 1-3; und
Transplantieren des Spenderorgans in einen Empfänger, worin die implantierbare Vorrichtung das Risiko der Abstoßung des Spenderorgans im Vergleich zu dem Risiko eines Spenderorgans, das ohne Kontakt mit der implantierbaren Vorrichtung transplantiert wurde, verringert.

8. S-Nitrosyliertes Lipid oder S-Nitrosyliertes Phospholipid, wie S-Nitrosyliertes 1,2-Dipalmitoyl-sn-glycero-3-phosphothioethanol (DPPTE), zur Verwendung in einem Verfahren zur Transplantation eines Organs in einen Empfänger, das Verfahren umfassend:
Herstellen einer implantierbaren Vorrichtung, die ein Organ ist; worin die implantierbare Vorrichtung eine Vorrichtung gemäß einem der Ansprüche 1-3 ist; und Transplantieren des Organs in einen Empfänger.

## Revendications

1. Dispositif implantable comprenant une nanostructure de molécules réservoirs qui sont des réservoirs d'oxyde nitrique ;
dans lequel la molécule réservoir est un lipide S-Nitrosylé ou un phospholipide S-Nitrosylé tel que le 1,2-dipalmitoyl-sn-glycero-3-phosphothioethanol (DPPTE) S-Nitrosylé ; et
dans lequel la nanostructure présente un noyau d'or et une bicouche ou une monocouche lipidique.

2. Dispositif implantable selon la revendication 1, comprenant en outre une apolipoprotéine, facultativement l'apolipoprotéine A-I (apoA-I).

3. Dispositif implantable selon la revendication 1 ou la revendication 2, dans lequel le dispositif implantable est un stent, et facultativement un stent à élution de médicament.

4. Nanostructure de molécules réservoirs qui sont des réservoirs d'oxyde nitrique pour utilisation dans un procédé d'administration de NO chez un sujet souffrant d'un trouble médié par le NO, le procédé comprenant :
l'administration par injection, à travers un cathéter ou sur un dispositif implantable, de la nanostructure de molécules réservoirs,
dans laquelle les molécules réservoirs sont un lipide S-Nitrosylé ou un phospholipide S-Nitrosylé tel que le 1,2-dipalmitoyl-sn-glycero-3-phosphothioethanol (DPPTE) S-Nitrosylé ;
et dans laquelle la nanostructure présente un noyau d'or et une bicouche ou une monocouche lipidique.

5. Nanostructure de molécules réservoirs selon la revendication 4, dans laquelle le trouble médié par le NO est l'angiogenèse, l'ischémie-reperfusion, la resténose, l'angioplastie, l'athérosclérose, un dérèglement de la tension artérielle ou un rejet de greffe après transplantation.

6. Nanostructure de molécules réservoirs selon la revendication 4 ou la revendication 5, dans laquelle la nanostructure comprend en outre une apolipoprotéine, facultativement l'apolipoprotéine A-I (apoA-I).

7. Lipide S-Nitrosylé ou phospholipide S-Nitrosylé tel que le 1,2-dipalmitoyl-sn-glycero-3-phosphothioethanol (DPPTE) S-Nitrosylé pour utilisation dans un procédé de transplantation d'un organe de donneur chez un sujet receveur, le procédé comprenant :
le prélèvement d'un organe de donneur ;
la mise en contact de l'organe du donneur avec le dispositif implantable selon l'une quelconque des revendications 1 à 3 ; et
la transplantation de l'organe de donneur sur un sujet receveur, dans lequel le dispositif implantable diminue le risque de rejet de l'organe de donneur par rapport au risque rencontré lorsqu'un organe de donneur est transplanté sans exposition au dispositif implantable.

8. Lipide S-Nitrosylé ou phospholipide S-Nitrosylé tel que le 1,2-dipalmitoyl-sn-glycero-3-phosphothioethanol (DPPTE) S-Nitrosylé pour utilisation dans un procédé de transplantation d'un organe chez un sujet receveur, le procédé comprenant :
la préparation d'un dispositif implantable qui est un organe ; dans lequel le dispositif implantable est un dispositif selon l'une quelconque des revendications 1 à 3 ; et la transplantation de l'organe sur un sujet receveur.
